# EUROPEAN PATENT APPLICATION

(11) **EP 3 777 534 A1**
(43) Date of publication of application: **17.02.2021**
(21) Application number: 20190771.4
(22) Date of filing: 25.03.2015
(51) Int. Cl.: A01N 1/02, A01N 63/00, C12N 5/071, C12N 5/00, C12N 15/00, A61K 38/47, A61K 35/28, C12N 5/0789, A61P 7/00

(54) **COMPOSITIONS AND METHODS FOR EX VIVO EXPANSION OF HUMAN HEMATOPOIETIC STEM/PROGENITOR CELLS**

(30) Priority: 26.03.2014 US 201461970787 P
(62) Divisional of application: 15769962.0
(71) Applicant: The Brigham & Women's Hospital, Inc., Boston, Massachusetts 02115 (US); Dana-Farber Cancer Institute, Inc., Boston, MA 02215 (US); National University of Singapore, Singapore 119077 (SG)
(72) Inventor: CHAI, Li, Sudbury, MA 01776 (US); TATETSU, Hiro, Brookline, MA 02445 (US); TENEN, Daniel Geoffrey, Singapore 267829 (SG); BRADNER, James Elliot, Weston, MA 02493 (US); FEDERATION, Alexander, Seattle, WA 98107 (US)
(74) Representative: Crease, Devanand John

(57) **Abstract**

Described herein are methods of genetically modifying a quantity of expanded hematopoietic cells, comprising providing a quantity of hematopoietic cells; culturing the quantity of hematopoietic cells in the presence of at least one histone deacetylase inhibitor (HDACi) and at least one growth factor to expand the hematopoietic cells; and contacting the expanded hematopoietic cells with a nucleic acid sequence. The nucleic acid sequence modifies the quantity of expanded hematopoietic cells. Also described are compositions comprising the quantity of expanded hematopoietic cells produced.

## Description

### GOVERNMENT SUPPORT

This invention was made with government support under NIH R01 HL092437 awarded by The National Institutes of Health. The government has certain rights in the invention.

### FIELD OF THE INVENTION

Described herein are methods and compositions that find use in the field of regenerative medicine as providing for efficient *ex vivo* expansion of hematopoietic cells and establishing therapeutic approaches in hematopoietic transplantation settings.

### BACKGROUND

Hematopoietic stem cells (HSCs) possess the unique capacity to self-renew and give rise to all types of mature cells within the blood and immune systems. These features have provided widespread clinical utility of HSC transplantation, although major sources of HSCs (human bone marrow, mobilized peripheral blood, and umbilical cord blood) remain limited as a donor supply. These problems are compounded by the need to seek out well-matched donors to recipients, thereby adding heightened complexity in ensuring a suitable and reliable supply of donor material. Further, patients suffering from disease resulting from a genetic mutation would benefit greatly from gene therapy techniques, wherein autologous material is manipulated *ex vivo,* and returned following correction of the corrected genetic defects. In various types of transplant categories, developing effective techniques for *ex vivo* expansion and genetic manipulation of HSCs could provide a ready, renewable resource outside of the existing donor infrastructure and establish new gene therapy techniques to treatment of diseases caused by genetic mutation.

It is well-understood that HSC self-renewal is regulated by both intrinsic and extrinsic signals. Despite great progress in understanding the molecular factors that support the self-renewal and differentiation of the hematopoietic system *in vivo,* less is known on how to modulate the factors that govern the self-renewal of HSCs and more primitive human hematopoietic stem/progenitor cells (HSPCs) ex *vivo.* Additionally, unlike in the case of embryonic stem cells (ESCs), expansion of HSC and/or HSPC in culture in general is at the expense of loss of "sternness". Emerging studies have suggested the possibility of establishing *ex vivo* culture conditions that lead to self-renewal and expansion of certain HSC populations, such as those expressing CD34 and CD90 markers, which appear to coincidence with marrow-repopulating potential. What is not known is whether extrinsic factors can be applied to further enhance expansion of HSC populations without loss of "sternness". It is further unclear whether such cells are receptive to genomic modification, which would greatly benefit the application of *ex vivo* cultured HSCs in therapeutic applications. Thus, there remains a need for methods for generating and expanding large numbers of human HSCs to increase the availability of cells for transplantation as a renewable therapeutic resource.

Described herein are methods and compositions which lead to the effective expansion of hematopoietic stem and progenitor cells *ex vivo.* Using combinations of small molecule drugs and cytokines/growth factors/grown factors targeting epigenetic status in cells, the Inventors discovered at least 10x expansion of HSPCs treated with a variety of compounds, such as TSA, MS-275 or DOT1 inhibitors. Importantly, these results were extendible across both human cord blood and peripheral mobilized stem/progenitor cells (PBSC). Further, following treatment with compounds such as TSA or MS 275, multiple genes such as HoxA9, HoxB4, GATA-2 and SALL4 implicated in HSPC function were unperturbed, and efficiency of genomic editing using lentivirus was greatly enhanced following treatment. These novel approaches could be potentially used therapeutically for expansion of cord blood HSPCs in transplantation settings as well as gene modifications such as gene therapy or other genomic editing/ engineering process such as Transcription Activator-Like Effector Nucleases (TALENs), Zinc Finger Nuclease (ZFNs) or Clustered Regularly Interspaced Short Palindromic Repeats (CRISPR).

### SUMMARY OF THE INVENTION

Described herein is a method of expanding hematopoietic cells, including providing a quantity of hematopoietic cells and culturing the quantity of hematopoietic cells in the presence of at least one small molecule and at least one growth factor, wherein the at least one small molecule and at least one growth factors are capable of expanding the hematopoietic cells. In another embodiment, the hematopoietic cells are hematopoietic stem cells (HSCs). In another embodiment, the hematopoietic cells are hematopoietic stem progenitor cells (HSPCs). In another embodiment, the hematopoietic cells are isolated from cord blood. In another embodiment, the hematopoietic cells are isolated from bone marrow. In another embodiment, the hematopoietic cells are isolated from peripheral blood. In another embodiment, the at least one small molecule is a histone deacetylase inhibitor (HDACi). In another embodiment, the HDACi includes one or more HDACi selected from trichostatin (TSA), DLS3, MS275, SAHA, and HDAC6 inhibitor161. In another embodiment, the at least one small molecule includes one or more small molecules selected from 5-Azacytidine, JQ1-S, JY1, UNC0638, JMJD3, JQ-EZ-05, SRI, DBZ, dmPGE2 and UM171. In another embodiment, the at least one growth factor includes one or more growth factors elected from stem cell factor (SCF), flt3 ligand (FL),interleukin-3 (IL3) and interleukin-6 (IL6).

Further described herein is a method of genomic editing including providing a quantity of hematopoietic cells, and culturing the quantity of hematopoietic cells in the presence of at least one small molecule and at least one growth factor, contacting the cells with one or more vectors, each vector encoding at least one selection cassette and/or at least one nuclease, and selecting for hematopoietic cells expressing the selection cassette and the nuclease, wherein cells expressing the selection cassette and the nuclease includ an edited genome. In another embodiment, the cells are hematopoietic stem cells (HSCs). In another embodiment, the pluripotent stem cells are hematopoietic stem progenitor cells (HSPCs). In another embodiment, the at least one nuclease is a Zinc Finger Nuclease (ZFN). In another embodiment, the at least one nuclease is a Transcription Activator-Like Effector Nuclease (TALENs). In another embodiment, the at least one nuclease is a CRISPR-associated protein (Cas) nuclease. In another embodiment, the one or more vector includes a vector encoding at least one selection cassette and at least one nuclease. In another embodiment, the quantity of hematopoietic cells are isolated from cord blood, bone marrow, or peripheral blood. In another embodiment, the method includesadministering the selected hematopoietic cells into a subject. In another embodiment, the hematopoietic cells are immunocompatible with the subject.

Further described herein is a quantity of cells produced by the method of genomic editing including providing a quantity of hematopoietic cells, and culturing the quantity of hematopoietic cells in the presence of at least one small molecule and at least one growth factor, contacting the cells with one or more vectors, each vector encoding at least one selection cassette and/or at least one nuclease, and selecting for hematopoietic cells expressing the selection cassette and the nuclease, wherein cells expressing the selection cassette and the nuclease includes an edited genome.

Also described herein is an *ex vivo* method of expanding hematopoietic cells, including obtaining a quantity of hematopoietic stem cells (HSCs) or hematopoietic stem progenitor cells (HSPCs) from cord blood, bone marrow, or peripheral blood, and expanding the HSCs or HSPCs by culturing the HSCs or HSPCs in the presence of at least one small molecule selected from trichostatin (TSA), DLS3, MS275, SAHA, HDAC6 inhibitor161, 5-Azacytidine, JQ1-S, JY1, UNC0638, JMJD3, JQ-EZ-05, SRI, DBZ, dmPGE2 and UM171, and at least one growth factor selected from stem cell factor (SCF), flt3 ligand (FL),interleukin-3 (IL3) and interleukin-6 (IL6), for a period of at least 48 hours, thereby expanding the HSCs or HSPCs In another embodiment, obtaining a quantity of HSPCs includes isolation of cells that express CD34+ and/or CD90+. In another embodiment, the period of at least 48 hours includes 72 hours, 96 hours, 120 hours, 144 hours, or 168 hours.

### BRIEF DESCRIPTION OF THE FIGURES

Exemplary embodiments are illustrated in referenced figures. It is intended that the embodiments and figures disclosed herein are to be considered illustrative rather than restrictive.

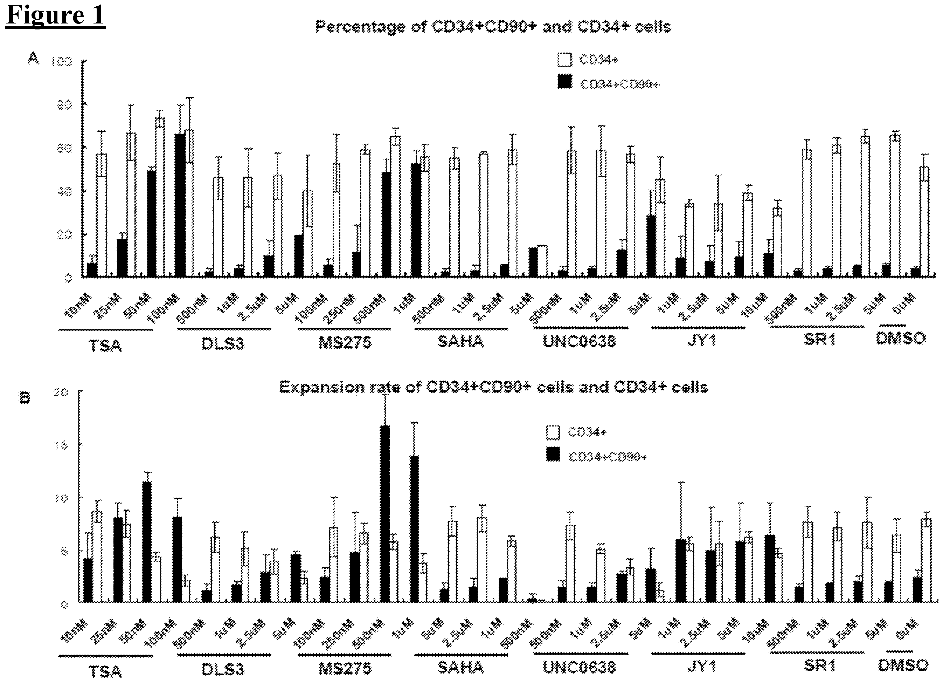

**Figure 1** **Screening with the compounds which increased CD34+CD90+ cells. (A)** Effect of the compounds on CD34 and CD90 expression following culture. Each value represents mean ± standard error of two independent experiments. (B) Absolute number of CD34+CD90+cells cultured with the compounds. Each value represents mean ± standard error of two independent experiments.

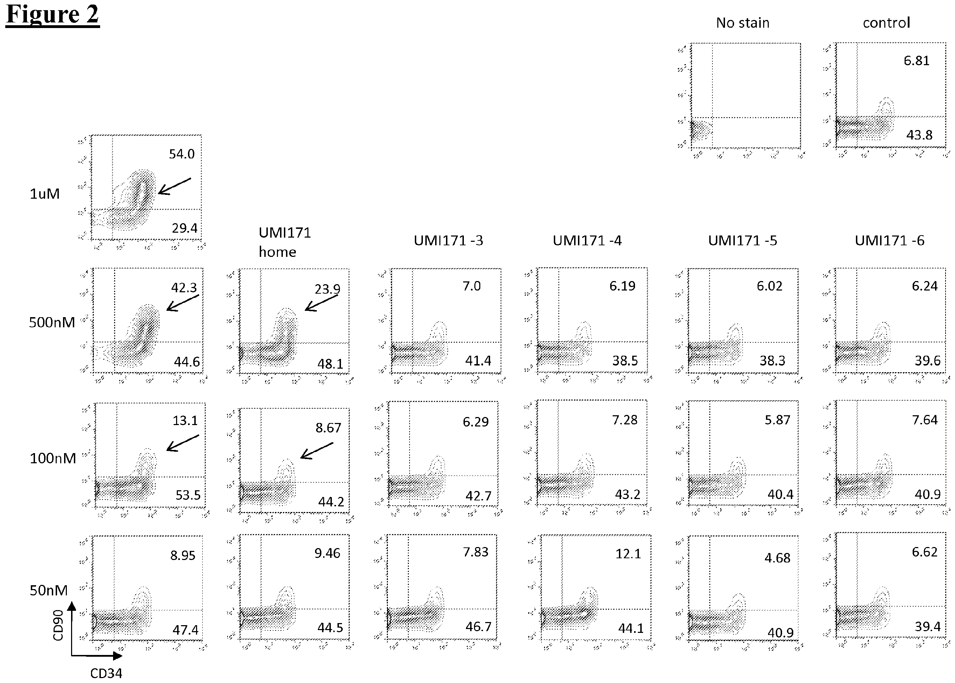

**Figure 2** **UM171 affects the division of CD34+ cells.** Total cell numbers are very similar between 50nM to 500nM (1uM) using commercial and synthesized (labeled home) UMI171 and its intermediate.

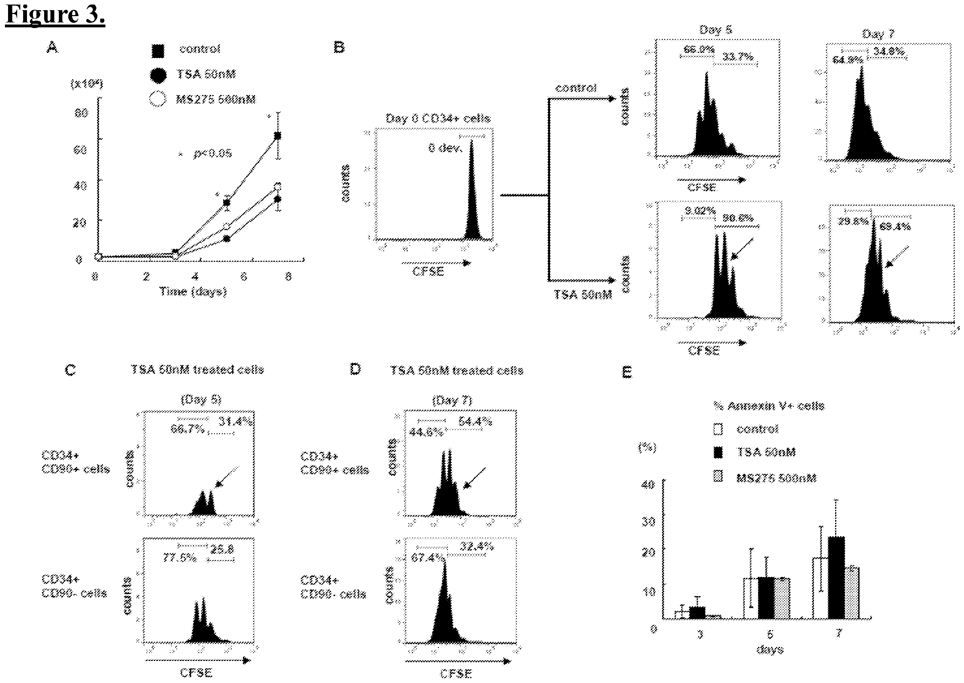

**Figure 3** **TSA affects the division of CD34+ cells. (A)** Cell growth of CD34+ cells cultured in the presence of cytokines/growth factors/growth factors with TSA, MS275 or DMSO. The data shown are the mean of three independent experiments. **(B)** CSFE-labeled CD34+ cells were cultured in the presence of cytokines/growth factors/growth factors with TSA or DMSO treatment for 7 days. The panel shows a representative (1 of 2 experiment) flow cytometric profile of CFSE fluorescence intensity after 5 and 7 days of culture. The arrow indicates the fraction of cells that have undergone less cell division. **(C)** The panel shows a representative flow cytometric profile of CFSE fluorescence intensity of CD34+CD90+ and CD34+CD90- cells after 5 of culture. **(D)** The panel shows a representative flow cytometric profile of CFSE fluorescence intensity of CD34+CD90+ and CD34+CD90- cells after 7 of culture. **(E)** Annexin V positive cell cultured in the presence of cytokines/growth factors/growth factors with TSA, MS275 or DMSO. The data shown are the mean of two independent experiments.

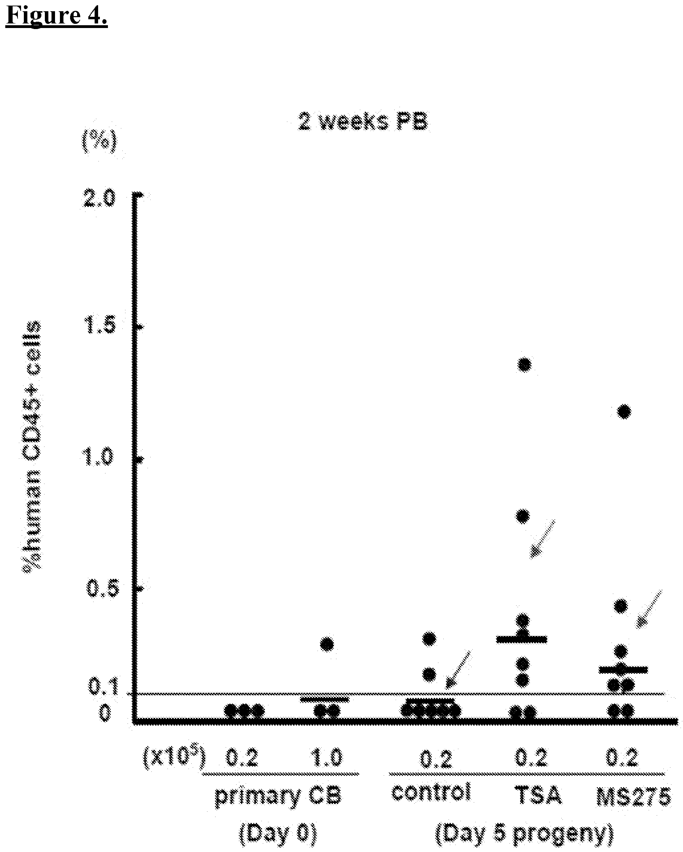

**Figure 4** **Treatment of CB cells with TSA enhances the marrow-repopulating potential.** A scatter plot showing the levels of human CD45+ cell engraftment in the PB of NSG mice 2 weeks after transplantation with the progeny of 2x 10⁴ CD34+ cells after culture with DMSO, TSA or MS275.

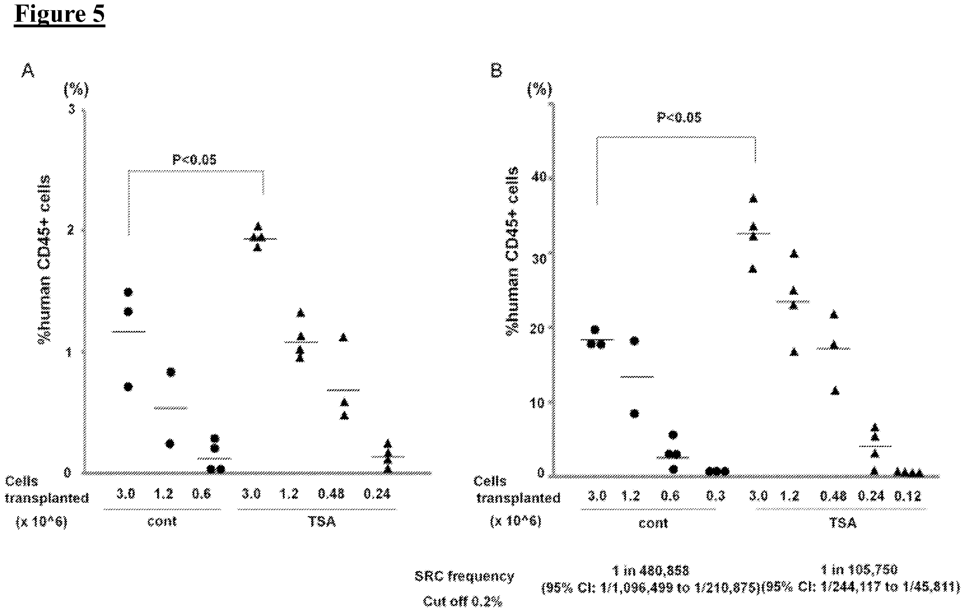

**Figure 5** **Treatment of HSPCs with TSA enhances the marrow-repopulating potential.** A scatter plot showing the levels of human CD45+ cell engraftment in the PB (A) and BM (B) of NSG mice 8 weeks after transplantation

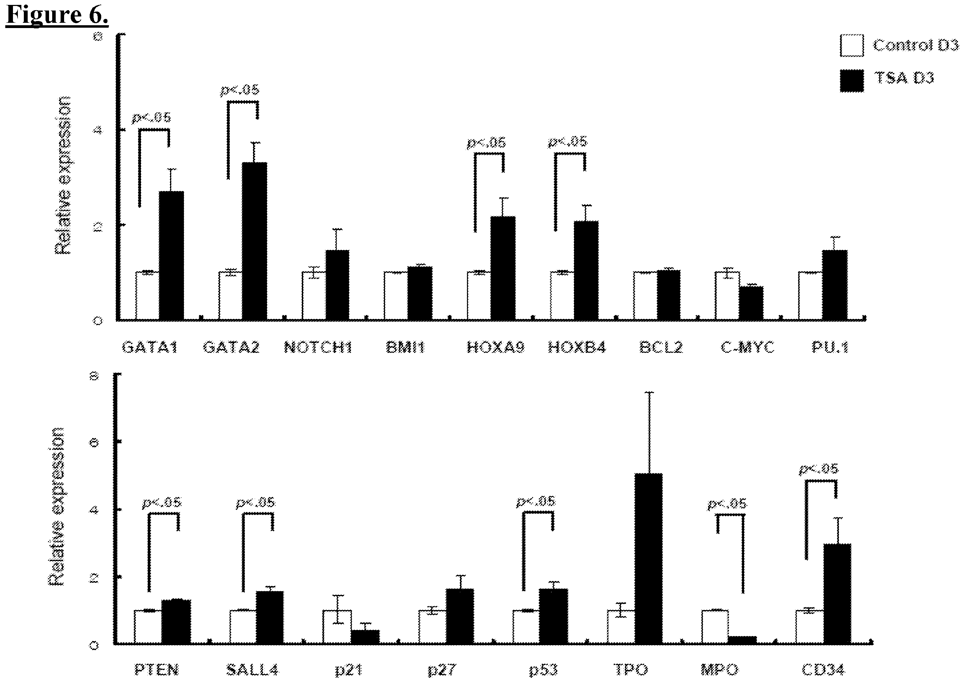

**Figure 6** **Treatment of CD34+ cells with TSA modulates expression of stem cell related genes.** Effects of wild type-peptide treatment on the relative transcript level of genes (GATA1, GATA2, NOTCH1, BMI1, HOXB4, C-MYC, PU.1, BCL2, P53, P21, P27, MPO, TPO and CD34 were measured by real-time quantitative PCR. Total RNA was extracted from cells obtained 3 days of culture in the presence of cytokines/growth factors/growth factors with TSA or DMSO. Relative mRNA levels TSA treated cells to DMSO treated cells were determined by real-time PCR. GAPDH was used as internal calibrator (control gene). Measurements were obtained in duplicate using 2 independent samples.

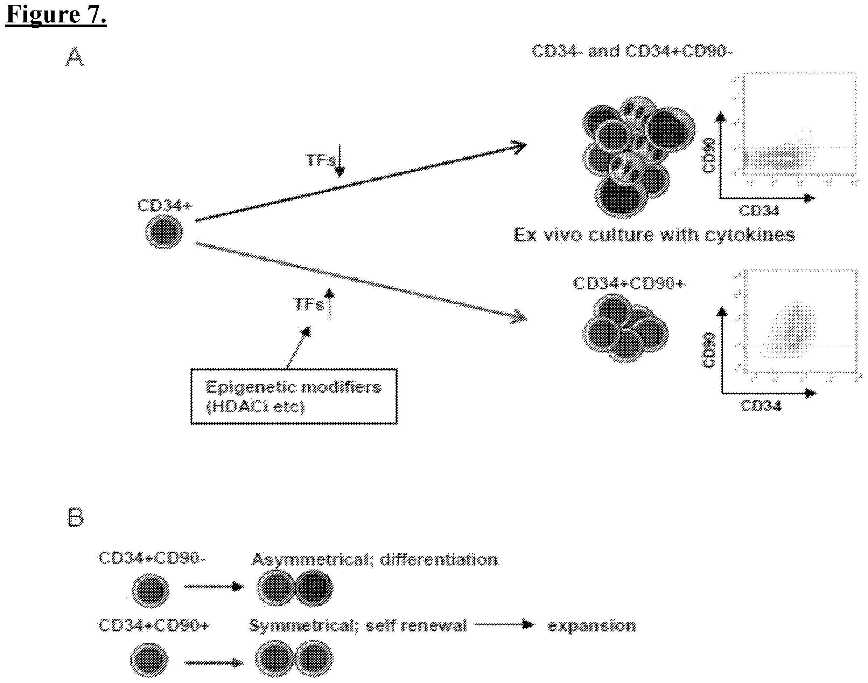

**Figure 7** **Schematic model of CD34+CD90+ expansion in human stem cells. (A)** HSPCs were tend to differentiate even though cells were under stimulated condition with cytokines/growth factors/growth factors containing media. Histone deacetylase inhibitor (HDACi) treated HSPCs were expanded rather than differentiation with cytokines/growth factors/growth factors containing media. **(B)** Schematic model on division models of CD34CD90+ cells.

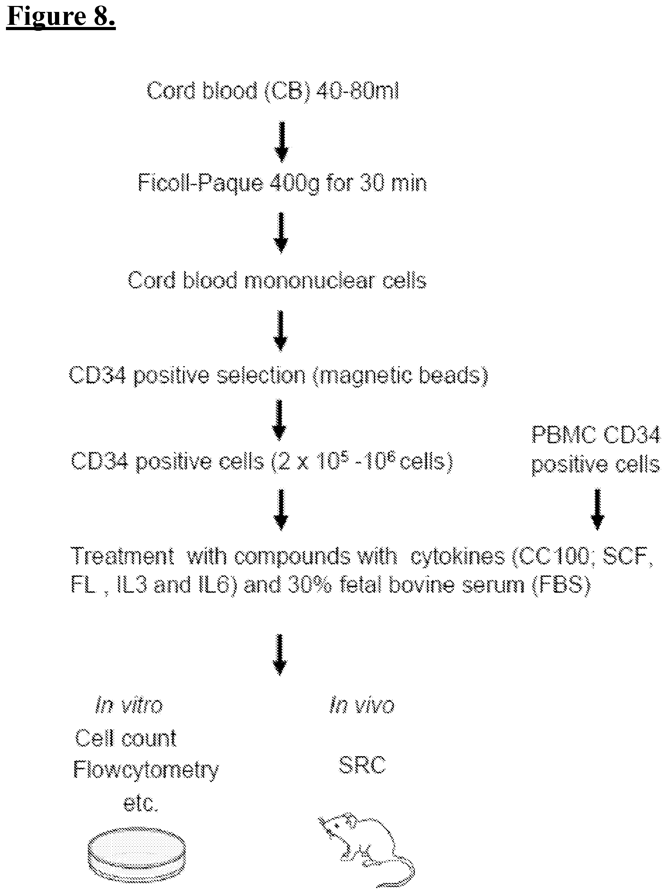

**Figure 8** **Diagram of the procedure analyzing CD34+CD90+ cells.** CB CD34+ cells were obtained by using Ficoll-Paque, CD34+ positive selection kit (magnetic beads). Cells were then treated with the peptide for one hour, followed by an addition of cytokines/growth factors/growth factors (CC100; SCF, FL, IL3 and IL6) and fetal bovine serum (FBS). Later, cells were analyzed *in vitro* and *in vivo.*

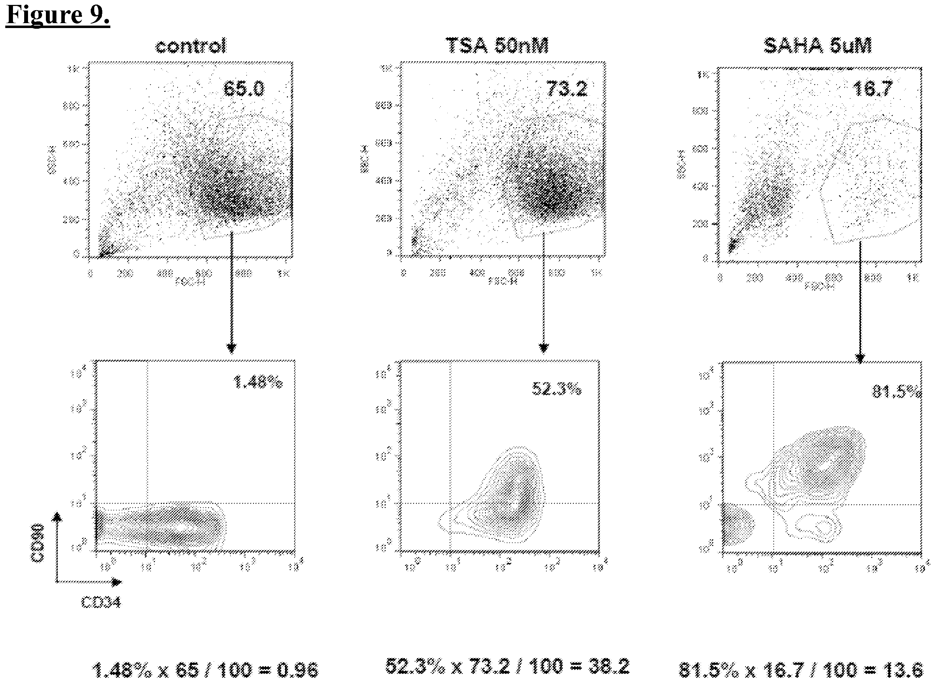

**Figure 9** **Examples of screening with the compounds which increased CD34+CD90+ cells.** Various examples of the properties of cells expanded following treatment with the described compounds.

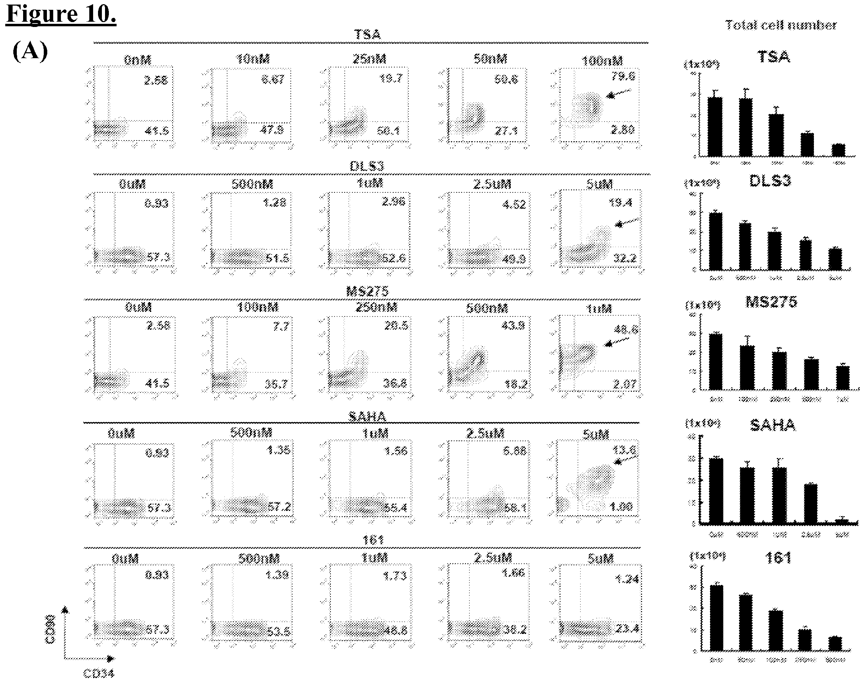

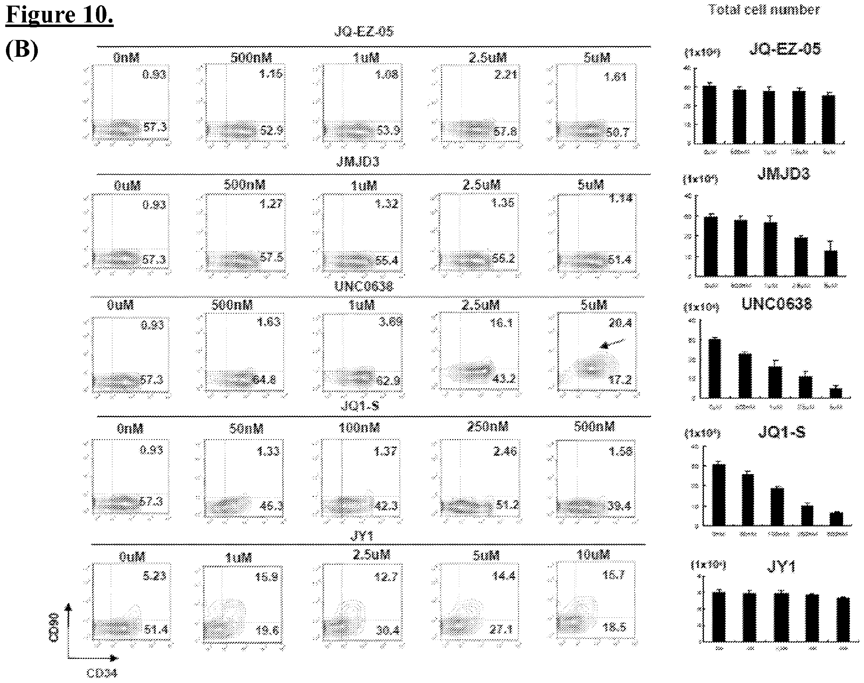

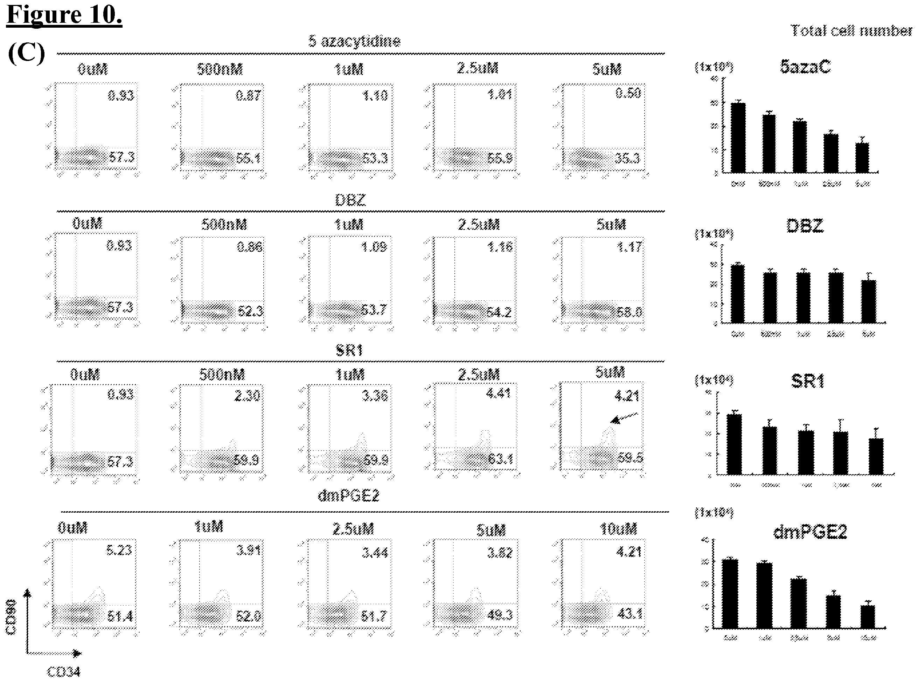

**Figure 10** **Screening with the compounds which increased CD34+CD90+ cells.** The Effect of the compounds on CD34 and CD90 expression following culture is shown on the left side. Results are representative data 1 of 2 experiments. Cell growth of CD34+ cells cultured in the presence of cytokines/growth factors/growth factors with compounds described on right side. The data shown are the mean of two independent experiments.

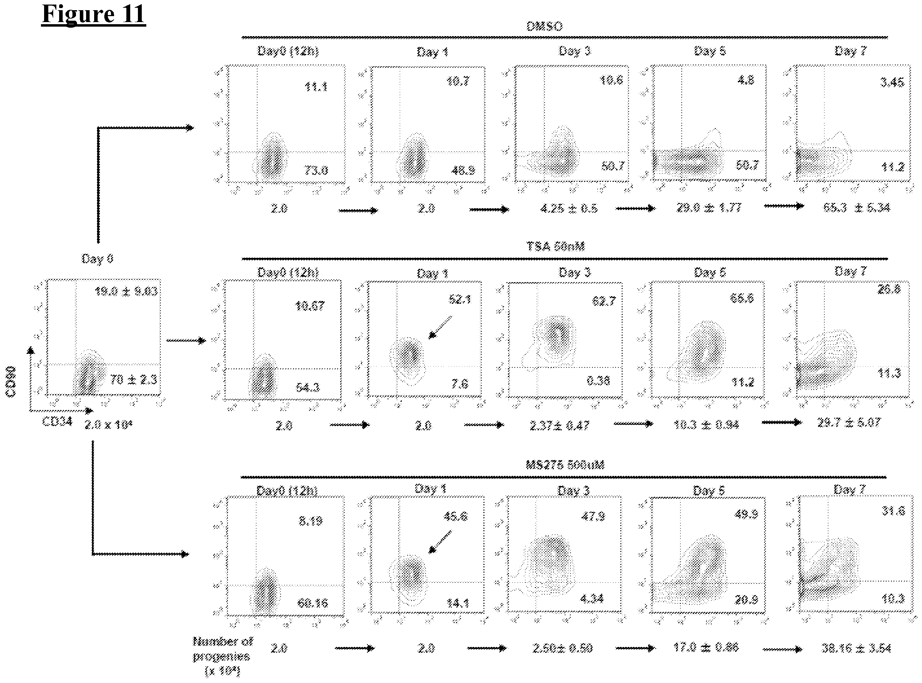

**Figure 11** **The change of CD34+CD90+ expression during ex vivo culture with TSA or MS275.** CD34+ cells were sorted and analyzed by flow cytometry. The number of progeny was described (mean ± SDV). The panel shows a representative flow cytometric profile.

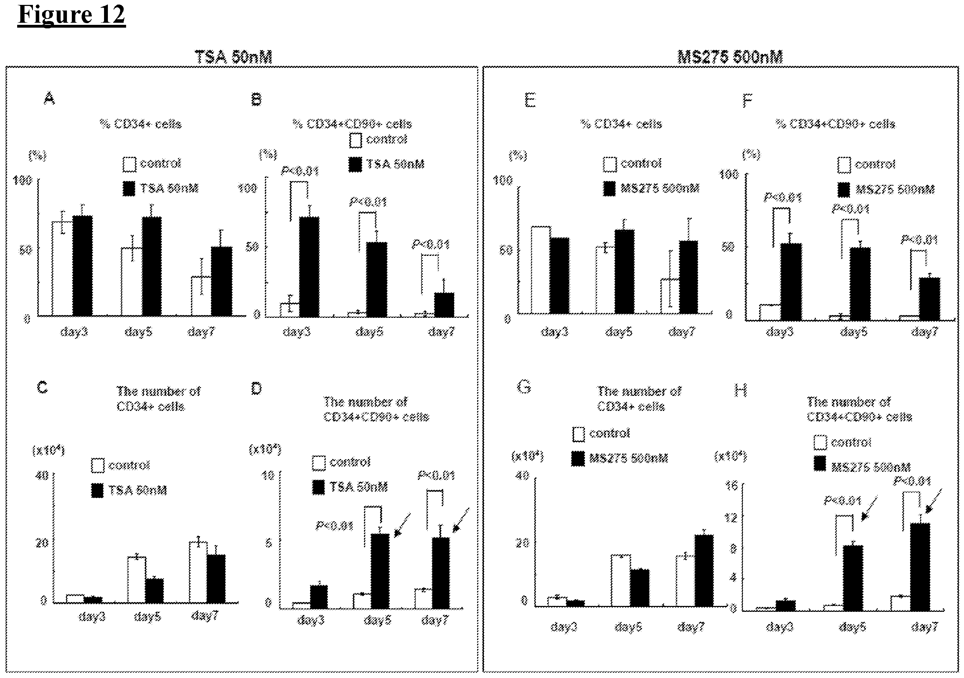

**Figure 12** **Treatment of CD34+ cells with TSA or MS275 expanded CD34+CD90+ cells during *ex vivo* culture. (A)** Percentage of CD34+ cells cultured with DMSO or TSA. **(B)** Percentage of CD34+CD90+ cells cultured with DMSO or TSA. **(C)** Absolute number of CD34+ cells cultured with DMSO or TSA. **(D)** Absolute number of CD34+CD90+ cells cultured with DMSO or TSA. **(E)** Percentage of CD34+ cells cultured with DMSO or MS275. **(B)** Percentage of CD34+CD90+ cells cultured with DMSO or MS275. **(C)** Absolute number of CD34+ cells cultured with DMSO or MS275. **(D)** Absolute number of CD34+CD90+ cells cultured with DMSO or MS275. Each value represents mean ± standard error of three independent experiments.

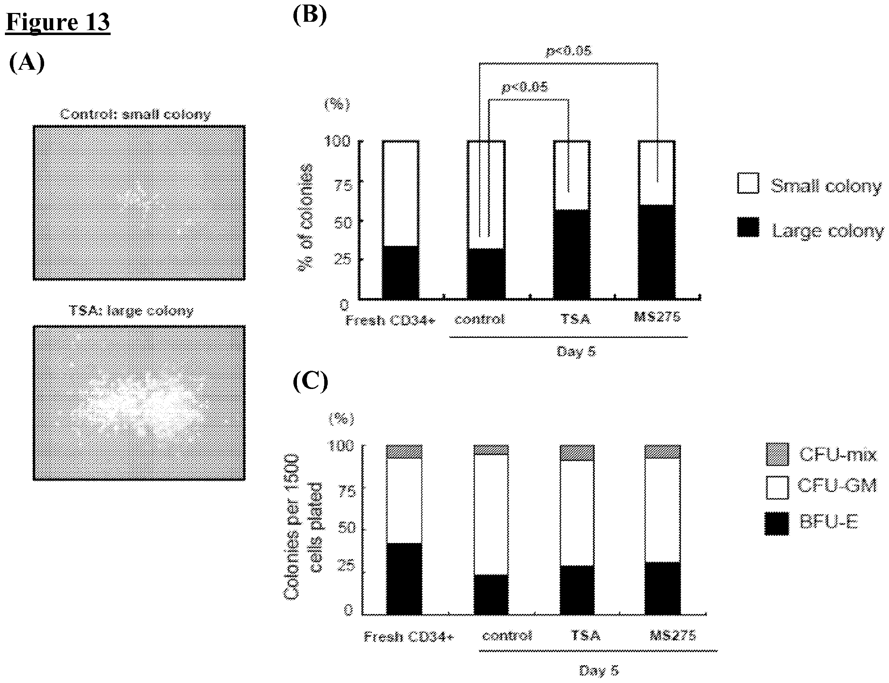

**Figure 13** **Characterization of treated cells.** (A) A large colony after culturing with TSA and a small colony after culturing with DMSO. Data shown is representative of three independent experiments. (B) Colonies were categorized into large (>50 clusters) and small (50-5 clusters). The white bar indicates small clusters. The black bar indicates large cluster. (C) The effect of wild type-peptide treatment on the number of the colony-forming cells (CFC). The CFU content of primary CD34+ cells, as well as treated with DMSO, TSA and MS275 were determined. Each value represents mean of three independent experiments.

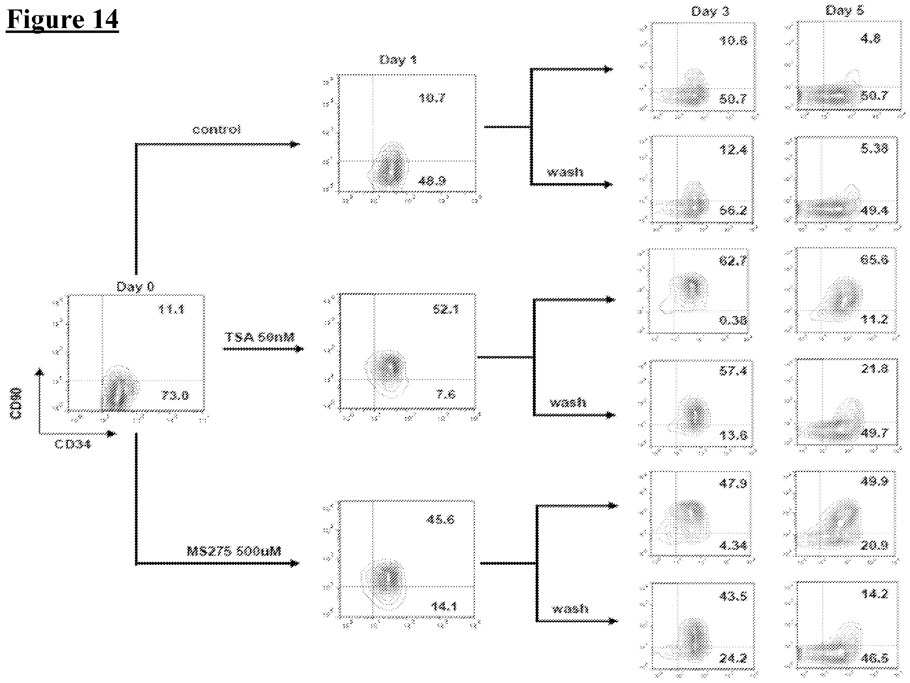

**Figure 14** **One day exposure to TSA or MS275 limit the expression of CD34+CD90+ cells.** Cells were cultured with DMSO (right upper), TSA (right middle) or MS275 (right lower) for 24 hours and then cell were washed with PBS or not washed. The CD34+CD90+ expression was evaluated on day 3 and day 5.

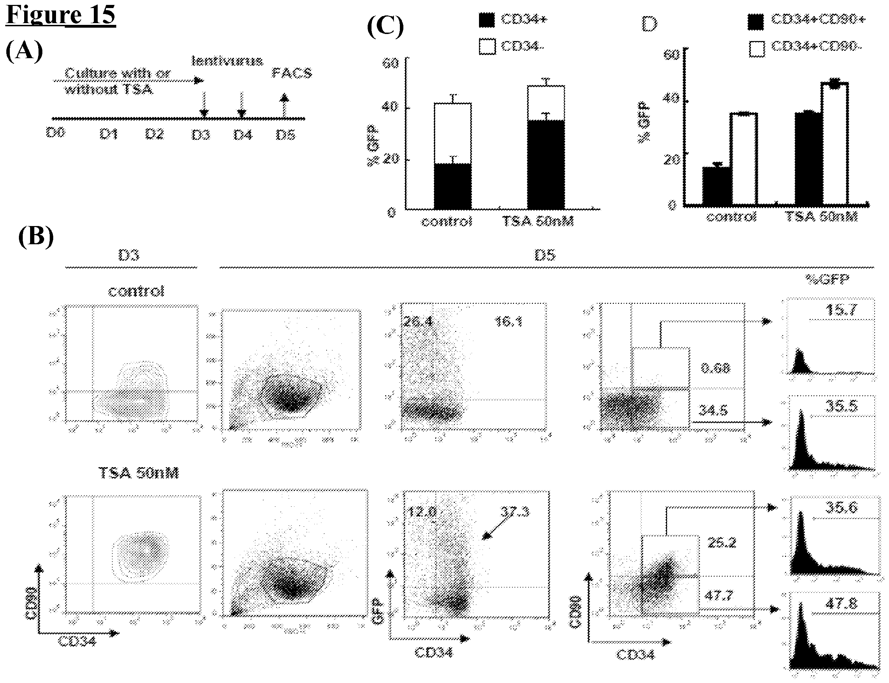

**Figure 15** **Treatment of PBMC CD34+ cells with TSA increased the efficiency of gene insertion approach. (A)** Cells were cultured with TSA or MS275 for 72 hours, then cell were infected in medium containing lentiviral particles. The culture medium was then removed and replaced with fresh media. **(B)** GFP expression was analyzed with CD34 and CD90 expression on day 5. **(C)** GFP positive cells with or without expression of CD34. (D) GFP expression on CD34+CD90+ and CD34+CD90- cells

### DETAILED DESCRIPTION OF THE INVENTION

All references cited herein are incorporated by reference in their entirety as though fully set forth. Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (2008); Singleton and Sainsbury, Dictionary of Microbiology and Molecular Biology 3rd ed., revised ed., J. Wiley & Sons (New York, NY 2006); Smith, March's Advanced Organic Chemistry Reactions, Mechanisms and Structure 7th ed., J. Wiley & Sons (New York, NY 2013); Singleton, Dictionary of DNA and Genome Technology 3rd ed., Wiley-Blackwell (November 28, 2012); and Green and Sambrook, Molecular Cloning: A Laboratory Manual 4th ed., Cold Spring Harbor Laboratory Press (Cold Spring Harbor, NY 2012), provide one skilled in the art with a general guide to many of the terms used in the present application. For references on how to prepare antibodies, see Greenfield, Antibodies A Laboratory Manual 2nd ed., Cold Spring Harbor Press (Cold Spring Harbor NY, 2013); Köhler and Milstein, Derivation of specific antibody-producing tissue culture and tumor lines by cell fusion, Eur. J. Immunol. 1976 Jul, 6(7):511-9; Queen and Selick, *Humanized immunoglobulins*, U. S. Patent No. 5,585,089 (1996 Dec); and Riechmann et al., Reshaping human antibodies for therapy, Nature 1988 Mar 24, 332(6162):323-7.

One skilled in the art will recognize many methods and materials similar or equivalent to those described herein, which could be used in the practice of the present invention. Indeed, the present invention is in no way limited to the methods described herein. For purposes of the present invention, the following terms are defined below.

"Administering" and/or "administer" as used herein refer to any route for delivering a pharmaceutical composition to a patient. Routes of delivery may include non-invasive peroral (through the mouth), topical (skin), transmucosal (nasal, buccal/sublingual, vaginal, ocular and rectal) and inhalation routes, as well as parenteral routes, and other methods known in the art. Parenteral refers to a route of delivery that is generally associated with injection, including intraorbital, infusion, intraarterial, intracarotid, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal. Via the parenteral route, the compositions may be in the form of solutions or suspensions for infusion or for injection, or as lyophilized powders.

"Modulation" or "modulates" or "modulating" as used herein refers to upregulation (i.e., activation or stimulation), down regulation (i.e., inhibition or suppression) of a response or the two in combination or apart.

"Pharmaceutically acceptable carriers" as used herein refer to conventional pharmaceutically acceptable carriers useful in this invention.

"Promote" and/or "promoting" as used herein refer to an augmentation in a particular behavior of a cell or organism.

"Subject" as used herein includes all animals, including mammals and other animals, including, but not limited to, companion animals, farm animals and zoo animals. The term "animal" can include any living multi-cellular vertebrate organisms, a category that includes, for example, a mammal, a bird, a simian, a dog, a cat, a horse, a cow, a rodent, and the like. Likewise, the term "mammal" includes both human and non-human mammals.

"Therapeutically effective amount" as used herein refers to the quantity of a specified composition, or active agent in the composition, sufficient to achieve a desired effect in a subject being treated. A therapeutically effective amount may vary depending upon a variety of factors, including but not limited to the physiological condition of the subject (including age, sex, disease type and stage, general physical condition, responsiveness to a given dosage, desired clinical effect) and the route of administration. One skilled in the clinical and pharmacological arts will be able to determine a therapeutically effective amount through routine experimentation.

"Treat," "treating" and "treatment" as used herein refer to both therapeutic treatment and prophylactic or preventative measures, wherein the object is to prevent or slow down (lessen) the targeted condition, disease or disorder (collectively "ailment") even if the treatment is ultimately unsuccessful. Those in need of treatment may include those already with the ailment as well as those prone to have the ailment or those in whom the ailment is to be prevented.

**Properties of Hematopoietic Stem Cells (HSCs) and Clinical Applications.** Hematopoietic stem cells (HSCs) and their primitive human hematopoietic stem/progenitor cells (HSPCs) counterparts possess the unique capacity to self-renew and give rise to mature cells within the blood and immune systems. Although hematopoietic stem cell (HSC) self-renewal divisions *in vitro* clearly occur, induction of self-renewal *in vitro* has been difficult. Even after several decades of research, the quest for factors that stimulate self-renewal *in vitro* is still continuing. HSC and HSPCs self-renewal is regulated by both intrinsic and extrinsic signals. Several factors have been identified whose action is associated with HSC and HSPCs self-renewal, including Notch ligands, Wnt3a, Angiopoietin-like proteins, Prostaglandin E2, Pleiotrophin and SALL4 and homeobox protein B4. Despite great progress in understanding the molecular factors that support the self-renewal and differentiation of the hematopoietic system *in vivo,* less is known on how to modulate the factors that govern the self-renewal of HSCs and HSPCs *in vitro* and *ex vivo.*

Of great interest scientifically is the wide versatility of HSC and HSPCs as obtainable from adult peripheral blood (PB), bone marrow, cord blood (CB), but also generated *in vitro* from pluripotent stem cells (PSCs). While a great deal of study is necessary to fully confirm cells from each of these sources are identical, or at least highly similar, what is clear is that they would all benefit greatly from techniques promoting their self-renewal and expansion capability. First, demonstration of such capability would help to functionally characterize differences and similarities. Second, enhanced production of these cell populations would allow for their further study, and help to realize production of therapeutically relevant numbers of cells has been difficult to achieve.

Of note is that in clinical settings, an increase in the utilization of CD transplantation has been observed in recent years, thereby presenting an attractive area for further investigation for *ex vivo* expansion. However, the use of CB as a hematopoietic stem cell (HSC) source is limited by the number of HSCs, and possible populations of primitive HSPCs, contained in the graft. To improve outcomes and extend applicability of CB transplantation, one potential solution is *ex vivo* expansion of CB. It is also crucial for *in vitro* manipulation of HSCs in gene therapy approaches.

While cell surface markers such as CD38, CD45RA, CD49f and CD90 have been investigated to enrich freshly isolated HSCs and possibly, primitive HSPCs, in addition to CD34, it is also believed that cells with co-expression of CD34 and CD90 are responsible for marrow-repopulating potential following *ex vivo* culture. For this reason, it is of interest to understand if CD34+CD90+ can determine the expansion of marrow-repopulation cells.

**Role of Epigenetic Status in Fate Specification.** Importantly, if HSC and HSPC fate is governed by transcription factors (TFs), which are down-regulated during *ex vivo* expansion, TFs could play a role in maintain the HSC and HSPC identity by "bookmarking" the epigenetic memories during cell division. The combination of cytokines/growth factors/growth factors and epigenetic modifiers could therefore expand HSC and HSPC or favor self-renewal during a cell division, which is thought to be mediated, at least in part by the maintained TFs.

In view of the promising candidate population of CD34+CD90+ cells for expansion capability, described herein are human HSC self-renewal properties focusing on human CD34+CD90+ cells to evaluate the possibility *ex vivo* culture conditions sufficient to maintain and expand the HSC and HSPC cells. If such methods can be established, one can further enhance the efficacy of gene therapy approaches by targeted, specific modification of the genetic information - or genome - of HSPCs, which includes traditional gene therapy approach, or Transcription Activator-Like Effector Nucleases (TALENs), Zinc Finger Nuclease (ZFNs) or Clustered Regularly Interspaced Short Palindromic Repeats (CRISPR)-associated endonuclease protein (Cas) system. Establishing *ex vivo* expansion methods, along with improved methods for genomic editing would provide a renewable resource of immunologically variable transplant material, thereby meeting a pressing and urgent clinical need that has long been unfulfilled.

**Genomic Editing.** In view of the need to establish immunological compatibility of transplant material, genome engineering is powerful tool for regenerative medicine applications. Site-specific chromosomal integration can target desired nucleotide changes, including introducing therapeutic gene cassettes in safe landing sites within chromosomes, disrupting the coding or non-coding regions of specific alleles and correcting the genetic mutations to reverse the disease phenotype. Recently developed methodologies employing nucleases such as Zinc Finger Nuclease (ZFNs), Transcription Activator-Like Effector Nucleases (TALENs) and Clustered Regularly Interspaced Short Palindromic Repeats (CRISPR)-associated endonuclease protein (Cas) system, can make double strand break to increase frequency of gene targeting.

Briefly, genome editing using tools such as ZFNs can be based on the introduction of a site-specific DNA DSB into the locus of interest. Key to this process is the cellular repair mechanism for efficiently repair DSBs via the homology-directed repair (HDR), or non-homologous end joining (NHEJ) pathways. The mechanisms of these DNA repair pathways can generate defined genetic outcomes. For example, NHEJ repair, can rapidly and efficiently ligate two broken ends, providing opportunity for the gain or loss of genetic information, allowing small insertions and/or deletions at the site of the break, thereby allowing disruption of a target gene. Alternatively, specifically-designed homologous donor DNA provided in combination with ZFNs, this template can result in gene correction or insertion.

One example of genomic editing in HSCs for clinical applications include establishing cells that are immunologically compatible with a recipient, as the number of people in need of a cell or tissue transplant, such as an HSC transplant, is far greater than the available supply of cells and tissues suitable for transplantation. HLAs are proteins on a cell's surface that help the immune system identify the cells as either self or non-self (foreign or from outside the body) and are encoded by clusters of genes that form a region located on human chromosome 6 known as the Major Histocompatibility Complex, or MHC, in recognition of the important role of the proteins encoded by the MHC loci in graft rejection (accordingly, the HLA proteins are also referred to as MHC proteins). For a patient who does not have a matched, related donor, a search through donor banks may provide a person with matching HLA types although obtaining a good match between the MHC proteins of a recipient and those of the transplant is frequently impossible. Generation and manipulation of HSCs to provide cells with wide immunological compatibility with larger groups of patient populations, such as establishing homo- or hemi-zygous HLA cells, would greatly aid availability of transplant material. Similar needs exist in available blood for transfusion, as is especially true for patients with unique blood types, patients who are Rh+. Generation of hematopoietic cells that are type O and Rh negative can be universally used for blood transfusion. The ability to generate and manipulate HSCs will greatly benefit the treatment and management of human disease. An additional example includes gene therapy techniques for patients suffering from disease resulting from a genetic mutation. Enhanced opportunities for genomic editing would fully realize the goal of altering autologous donor material *ex vivo,* to be returned following correction of the corrected genetic defects.

What presently limits gene targeting methods are the extremely low frequency at which HR occurs in most cells. Various ZFNs, TALENs and the Cas systems generate site specific DSBs within the genome specifically in the target locus can facilitate gene targeting by increasing the frequency of HR by several multitudes compared to conventional methods of HR induced targeting that were used in mice and yeast. These novel technologies hold great promise for genome engineering although generating the specific ZFNs, TALENs or Cas system still remains technically very challenging and time-consuming thus limiting their wide spread use by researchers. These genome engineering approaches are still limited by low frequency of HR events, especially in human pluripotent stem cells (PSCs), HSPCs, and HSCs.

As further described herein, it was observed that the combinations of small molecule drugs and cytokines/growth factors/growth factors can be applied in potential in expansion of purified CD34+CD90+ human cord blood or peripheral mobilized stem/progenitor cells (PBSC) CD34+ cells including treatment with drug compounds as such TSA, MS-275 or DOT1 inhibitors. When compared control-treated groups, drug compound treated CD34+ cells yielded an 11 to 16 fold expansion of primitive HSPCs (CD34+CD90+) *in vitro.* TSA also promoted the lentiviral transduction for CD34+ cells *ex vivo.* Furthermore, following TSA or MS 275 drug treatment, the Inventors observed increased expression of HoxA9, HoxB4, GATA-2 and SALL4 genes implicated in HSPC function. These results demonstrate that epigenetic modifiers can influence HSPC function and expression of a number of critical genes.

Described herein is a method of expanding hematopoietic cells, including: (a) providing a quantity of hematopoietic cells, and (b) culturing the quantity of hematopoietic cells in the presence of at least one small molecule and at least one growth factor, wherein the at least one small molecule and at least one growth factors are capable of expanding the hematopoietic cells. In a different embodiment, the hematopoietic cells are hematopoietic stem cells (HSCs). In a different embodiment, the hematopoietic cells are hematopoietic stem progenitor cells (HSPCs). Optionally, the HSPCs are cells that express CD34+ and/or CD90+. In other embodiments, the hematopoietic cells, such as hematopoietic stem cells (HSCs) or hematopoietic stem progenitor cells (HSPCs), express one or more markers, including Lin-, CD34+, CD38-, CD90+, CD45RA-. In other embodiments, the hematopoietic cells, such as hematopoietic stem cells (HSCs) or hematopoietic stem progenitor cells (HSPCs), express HOXB4, BMI1, GATA2, p21, p27, c-myc and MPO.

In a different embodiment, the hematopoietic cells are isolated from cord blood. In a different embodiment, the hematopoietic cells are isolated from bone marrow. In a different embodiment, the hematopoietic cells are isolated from peripheral blood. In a different embodiment, the at least one small molecule is a histone deacetylase inhibitor (HDACi). In a different embodiment, the HDACi includes one or more HDACi selected from the group of: trichostatin (TSA), DLS3, MS275, SAHA, and HDAC6 inhibitor161. In a different embodiment, the at least one small molecule includes one or more small molecules selected from the group of: 5-Azacytidine, JQ1-S, JY1, UNC0638, JMJD3, JQ-EZ-05, SRI, DBZ, and dmPGE2, and UM171. In various embodiments, concentrations of these small molecules can include about 2, 3, 4, 5, 6, 7, 8, 9, 10 nM or more, or about 25, 50, 100, 150, 250, 500 nM or more, or about 500, 600, 700, 800, 900, 1000 nM or more, or about 1, 2, 3, 4, 5, 6, 7, 8, 9, or 10 µM or more, with a variety of examples shown in Figure 1. In a different embodiment, the at least one growth factor includes one or more growth factors elected from the group of: stem cell factor (SCF), flt3 ligand (FL), interleukin-3 (IL3) and interleukin-6 (IL6). In various embodiments, concentrations of these growth factors can include, for example, about 100 ng/ml FL, about 100 ng/ml SCF, about 20 ng/ml IL-3 and about 20 ng/ml IL-6. In other embodiments, these growth factors can include concentrations of about 200-500 ng/ml FL, about 20-1000 ng/ml SCF, about 5-500 ng/ml IL-3 and about 5-500 ng/ml IL-6. In other embodiments, growth factors include early stage cytokines/growth factors/growth factors such as, EPO, TPO, FL, VEGF, BMPs like BMP2, BMP4 and BMP7, GM-CSF, G-CSF, and HOXB4. In certain embodiments, hematopoietic cells, such as hematopoietic stem cells (HSCs) or hematopoietic stem progenitor cells (HSPCs) are cultured in the presence of methylcellulose medium to promote hemangioblast growth. In a different embodiment, culturing the quantity of hematopoietic cells in the presence of at least one small molecule and at least one growth factor, wherein the at least one small molecule and at least one growth factors is for a period of at least 48 hours 72 hours, 96 hours, 120 hours, 144 hours, or 168 hours.

Further described herein, the methods also relate to *in vitro* expansion of hematopoietic cells, such as hematopoietic stem cells (HSCs) or hematopoietic stem progenitor cells (HSPCs), to generate large quantities useful for a variety of commercial and clinical applications. This includes, for example, at least 10,000, 100,000, or 500,000 cells). In certain embodiments, the cell preparations comprise at least 1 X 10⁶ hematopoietic cells. In other embodiments, the cell preparations comprise at least 2 X 10⁶ hematopoietic cells and in further embodiments at least 3 X 10⁶ hematopoietic cells. In still other embodiments, the cell preparations comprise at least 4 X 10⁶ hematopoietic cells.

The present invention relates to a solution, a preparation, and a composition comprising between 10,000 to 4 million or more human hematopoietic cells. The number of hematopoietic cells in such a solution, a preparation, and a composition may be any number between the range of 10,000 to 4 million, or more. This number could be, for example, 20,000, 50,000, 100,000, 500,000, 1 million, etc. The invention further relates to methods of producing, storing, and distributing hematopoietic cells. In certain embodiments, the invention provides for the use of the hematopoietic cells in the manufacture of a medicament to treat a condition in a patient in need thereof. Alternatively, the invention provides the use of the cell cultures in the manufacture of a medicament to treat a condition in a patient in need thereof. The invention also provides the use of the pharmaceutical preparations in the manufacture of a medicament to treat a condition in a patient in need thereof.

In various embodiments, expanding human hematopoietic cells includes hematopoietic cells obtained from any source, including cord blood from placenta or umbilical tissue, peripheral blood, bone marrow, embryonic stem cells, induced pluripotent stem cells, or other tissue or by any other means known in the art. In certain embodiments, hematopoietic cells, such as hematopoietic stem cells (HSCs) or hematopoietic stem progenitor cells (HSPCs), can be further differentiated to hematopoietic cells including, but not limited to blood cells such as megakaryocytes, platelets, red blood cells or immune cells such as natural killer or dendritic cells. Such cells may be used in transplantations or transfusions. The methods of this invention allow for ex vivo expansion of, hematopoietic cells, such as hematopoietic stem cells (HSCs) or hematopoietic stem progenitor cells (HSPCs), for a variety of therapies in which autologous tissue transplantations can serve to treat a disease and/or condition. In other instances, heterologous tissue transplantation can serve to treat the disease and/or condition, including for example, familial relatives of a subject. In some instances, this includes, for example, use of gene therapy.

Further described herein is a method of genomic editing including: (a) providing a quantity of hematopoietic cells, and (b) culturing the quantity of hematopoietic cells in the presence of at least one small molecule and at least one growth factor, (c) contacting the cells with one or more vectors, each vector encoding at least one selection cassette and/or at least one nuclease; and selecting for hematopoietic cells expressing the selection cassette and the nuclease, wherein cells expressing the selection cassette and the nuclease include an edited genome.

In a different embodiment, the cells are hematopoietic stem cells (HSCs). In a different embodiment, the pluripotent stem cells are hematopoietic stem progenitor cells (HSPCs). Optionally, the HSPCs are cells that express CD34+ and/or CD90+. In other embodiments, the hematopoietic cells, such as hematopoietic stem cells (HSCs) or hematopoietic stem progenitor cells (HSPCs), express one or more markers, including Lin-, CD34+, CD38-, CD90+, CD45RA-. In other embodiments, the hematopoietic cells, such as hematopoietic stem cells (HSCs) or hematopoietic stem progenitor cells (HSPCs), express HOXB4, BMI1, GATA2, p21, p27, c-myc and MPO. In a different embodiment, the at least one nuclease is a Zinc Finger Nuclease (ZFN). In a different embodiment, the at least one nuclease is a Transcription Activator-Like Effector Nuclease (TALENs). In a different embodiment, the at least one nuclease is a CRISPR-associated protein (Cas) nuclease. In a different embodiment, the one or more vector includes a vector encoding at least one selection cassette and at least one nuclease. In a different embodiment, the quantity of hematopoietic cells is isolated from cord blood, bone marrow, or peripheral blood.

Also described herein is a method of genomic editing including: (a) providing a quantity of hematopoietic cells, and (b) culturing the quantity of hematopoietic cells in the presence of at least one small molecule and at least one growth factor, (c) contacting the cells with one or more vectors, each vector encoding at least one selection cassette and/or at least one nuclease, and selecting for hematopoietic cells expressing the selection cassette and the nuclease, wherein cells expressing the selection cassette and the nuclease include an edited genome, and (d) administering the selected hematopoietic cells into a subject. In a different embodiment, the hematopoietic cells are immunocompatible with the subject.

Also described herein is a quantity of cells produced by a method of genomic editing including: (a) providing a quantity of hematopoietic cells, and (b) culturing the quantity of hematopoietic cells in the presence of at least one small molecule and at least one growth factor, (c) contacting the cells with one or more vectors, each vector encoding at least one selection cassette and/or at least one nuclease, and selecting for hematopoietic cells expressing the selection cassette and the nuclease, wherein cells expressing the selection cassette and the nuclease include an edited genome.

Further described herein is an *ex vivo* method of expanding hematopoietic cells, including: (a) obtaining a quantity of hematopoietic stem cells (HSCs) or hematopoietic stem progenitor cells (HSPCs) from cord blood, bone marrow, or peripheral blood, and (b) expanding the HSCs or HSPCs by culturing the HSCs or HSPCs in the presence of: (i) at least one small molecule selected from the group of: trichostatin (TSA), DLS3, MS275, SAHA, HDAC6 inhibitor161, 5-Azacytidine, JQ1-S, JY1, UNC0638, JMJD3, JQ-EZ-05, SRI, DBZ, and dmPGE2; and (ii) at least one growth factor selected from the group of: stem cell factor (SCF), flt3 ligand (FL),interleukin-3 (IL3) and interleukin-6 (IL6), for a period of at least 48 hours, thereby expanding the HSCs or HSPCs. In various embodiments, concentrations of these growth factors can include, for example, about 100 ng/ml FL, about 100 ng/ml SCF, about 20 ng/ml IL-3 and about 20 ng/ml IL-6. In other embodiments, growth factors include early stage cytokines/growth factors/growth factors such as, EPO, TPO, FL, VEGF, BMPs like BMP2, BMP4 and BMP7, GM-CSF, G-CSF, and HOXB4. In certain embodiments, hematopoietic cells, such as hematopoietic stem cells (HSCs) or hematopoietic stem progenitor cells (HSPCs) are cultured in the presence of methylcellulose medium to promote hemangioblast growth. In other embodiments, these growth factors can include concentrations of about 200-500 ng/ml FL, about 20-1000 ng/ml SCF, about 5-500 ng/ml IL-3 and about 5-500 ng/ml IL-6. In a different embodiment, obtaining a quantity of HSPCs includes isolation of cells that express CD34+ and/or CD90+. In a different embodiment, the period of at least 48 hours includes 72 hours, 96 hours, 120 hours, 144 hours, or 168 hours.

Further described herein is a method of treating a subject for a disease or condition, including Also described herein is a method of genomic editing including: (a) obtaining a quantity of hematopoietic cells, such as hematopoietic stem cells (HSCs) or hematopoietic stem progenitor cells (HSPCs), from cord blood, bone marrow, or peripheral blood from a subject in need of treatment for a disease or condition, (b) expanding the quantity of hematopoietic cells by culturing in the presence of at least one small molecule and at least one growth factor, wherein the at least one small molecule and at least one growth factors are capable of expanding the hematopoietic cells, and (c) administering the hematopoietic cells into a subject. In a different embodiment, the hematopoietic cells are immunocompatible with the subject. In certain embodiments, prior to administering the hematopoietic cells into a subject, the cells are contacted with one or more vectors, each vector encoding at least one selection cassette and/or at least one nuclease, and selecting for hematopoietic cells expressing the selection cassette and the nuclease, wherein cells expressing the selection cassette and the nuclease include an edited genome. In certain embodiments, the hematopoietic stem cells, such as (HSCs) or hematopoietic stem progenitor cells (HSPCs), are obtained from a familial relative of the subject in need of treatment.

In a different embodiment, the hematopoietic cells are hematopoietic stem cells (HSCs). In a different embodiment, the hematopoietic cells are hematopoietic stem progenitor cells (HSPCs). Optionally, the HSPCs are cells that express CD34+ and/or CD90+. In other embodiments, the hematopoietic cells, such as hematopoietic stem cells (HSCs) or hematopoietic stem progenitor cells (HSPCs), express one or more markers, including Lin-, CD34+, CD38-, CD90+, CD45RA-. In other embodiments, the hematopoietic cells, such as hematopoietic stem cells (HSCs) or hematopoietic stem progenitor cells (HSPCs), express HOXB4, BMI1, GATA2, p21, p27, c-myc and MPO.

In a different embodiment, the at least one small molecule is a histone deacetylase inhibitor (HDACi). In a different embodiment, the HDACi includes one or more HDACi selected from the group of: trichostatin (TSA), DLS3, MS275, SAHA, and HDAC6 inhibitor161. In a different embodiment, the at least one small molecule includes one or more small molecules selected from the group of: 5-Azacytidine, JQ1-S, JY1, UNC0638, JMJD3, JQ-EZ-05, SRI, DBZ, dmPGE2 and UM171. In various embodiments, concentrations of these small molecules can include about 2, 3, 4, 5, 6, 7, 8, 9, 10 nM or more, or about 25, 50, 100, 150, 250, 500 nM or more, or about 500, 600, 700, 800, 900, 1000 nM or more, or about 1, 2, 3, 4, 5, 6, 7, 8, 9, or 10 µM or more, with a variety of examples shown in Figure 1. In a different embodiment, the at least one growth factor includes one or more growth factors elected from the group of: stem cell factor (SCF), flt3 ligand (FL),interleukin-3 (IL3) and interleukin-6 (IL6). In various embodiments, concentrations of these growth factors can include, for example, about 100 ng/ml FL, about 100 ng/ml SCF, about 20 ng/ml IL-3 and about 20 ng/ml IL-6. In other embodiments, these growth factors can include concentrations of about 200-500 ng/ml FL, about 20-1000 ng/ml SCF, about 5-500 ng/ml IL-3 and about 5-500 ng/ml IL-6. In other embodiments, growth factors include early stage cytokines/growth factors/growth factors such as, EPO, TPO, FL, VEGF, BMPs like BMP2, BMP4 and BMP7, GM-CSF, G-CSF, and HOXB4. In certain embodiments, hematopoietic cells, such as hematopoietic stem cells (HSCs) or hematopoietic stem progenitor cells (HSPCs) are cultured in the presence of methylcellulose medium to promote hemangioblast growth. In a different embodiment, culturing the quantity of hematopoietic cells in the presence of at least one small molecule and at least one growth factor, wherein the at least one small molecule and at least one growth factors is for a period of at least 48 hours 72 hours, 96 hours, 120 hours, 144 hours, or 168 hours.

### Example 1

### Isolation of CB and PBMC CD34+ cells and ex vivo culture

Fresh CB collections were obtained from Cell Manipulation Core Facility in Dana-Faber Cancer Institute (DF/HCC; Boston, MA) according to guidelines established by DF/HCC Institutional Review Board. CB cells were isolated by density centrifugation on Ficoll-Paque (Stem Cell Technologies, Vancouver, BC, Canada) and enriched using the CD34 positive cell isolation kit (Stem Cell Technologies). The purity of CD34+ cells ranged between 60% to 90%. cells were allotted to 2 × 10⁴ /well and incubated in IMDM containing 30% fetal bovine serum (FBS; GIBCO) supplemented with CC100 (SCF, FL, IL3 and IL6; Stem Cell Technologies).

Throughout the described methods, results are expressed as mean ± SDV when appropriate, unless otherwise indicated. Statistical differences were evaluated using the student t test with significance at p of 0.05 or less.

### Example 2

### CFU assay of CB CD34+ cells

Tubes of MethoCult® H4434 (Stem Cell Technologies) medium were thawed overnight in a 4°C refrigerator. The next morning CB CD34+ cells were prepared at 10X the final concentration required. Cell suspensions of 2000 cells per 0.3 mL were prepared. Cells were added to 3 mL of MethoCult® medium for duplicate cultures. A 16 gauge blunt-end needle attached to a 3 cc syringe was used to dispense the cells and MethoCult® medium into culture dishes. 1.1 mL of cells was dispensed per 35 mm dish. The two dishes were placed into a 100 mm petri dish and a third, uncovered 35 mm dish containing 3 mL of sterile water was also added. All 3 dishes were then covered within the 100 mm petri dish. The cells were incubated for 14-16 days at 37°C with 5% CO2 and ≥95% humidity. The BFU -E, CFU-GM and CFU-mix colonies were observed with bright field. CFUs were counted under the microscopy.

### Example 3

### Flow cytometric analysis

Cells were stained with antihuman CD34 monoclonal antibody conjugated to phycoerythrin (PE), or allophycocyanin (APC), antihuman CD90 conjugated fluorescein isothiocyanate (FITC) or APC, CD38 APC, CD 3 tri-color (TC), CD19 TC, CDllb TC, CD41 TC, CD45 FITC and mouse CD45 APC. The flow cytometry data were collected using FACS Calibur flow cytometer (Becton Dickinson, San Jose, CA, USA) or and analysed by using FLOWJO software.

### Example 4

### Carboxyfluorescein diacetate succinimidyl ester labeling to assess cell division

Primary CD34+ cells were labeled for 10 minutes at 37°C with 0.5 uM of carboxyfluorescein diacetate succinimidyl ester (CSFE; Invirtogen, NY, USA) in PBS. After 9 days of culture, cell were labeled with CD34 PE and analyzed for a progressive decline of fluoredcence intensity of CSFE using FACSCalibur flow cytometer.

### Example 5

### Engraftment of CD34+ cells in NSG mice

NSG (NOD.Cg-Prkdcscid I12rgtm1Wj1/SzJ, The Jackson Laboratory, ME, USA) mice were bred and maintained in the Children's Hospital Boston animal facility. All animal work has been approved by and done according to the guidelines of the IACUC under protocol 10-101832. The CB CD34+ cells treated with wild type-peptide and scrambled-peptide were injected intravenously via the tail vein into sub-lethally irradiated (220 rads) 8 to 16-week-old NSG mice. Engraftment was performed within 24 h after irradiation. Peripheral blood (PB) chimerism was monitored at 2 and 8 weeks post transplantation. Bone marrow (BM) chimerism was monitored at 8weeks post transplantation. These samples were subsequently subjected to flow cytometry analysis utilizing FITC-conjugated anti-human CD45 antibody and APC-conjugated anti-mouse CD45 antibody (eBiosciences, CA, USA). The percentage of human CD45+ cells was calculated as follows: % human CD45+ cells = No. human CD45+ cells/ (No. human CD45+ cells + No. mouse CD45+ cells) x 100. A threshold of 0.1% human CD45+ cells was established as a reliable predictor of positive engraftment.

### Example 6

### Real-time PCR

Inventors prepared RNA from cells treated with wild-type or scrambled peptide using TRIzol (Invitrogen). The Inventors synthesized cDNA using iScript cDNA Synthesis kit (Bio-Rad, CA, USA). To quantitate the level of mRNAs expression, The Inventors carried out polymerase chain reaction (PCR) amplification using iScript one-step RT-PCR kit with SYBER Green (Bio-Rad) and the PCR products were detected by use of SYBR green technology (ABI, Foster City, CA, USA). GAPDH was used as an endogenous control. All samples were run in duplicate. Thermal cycler conditions were 50°C for 2 minutes, 95°C for 10 minutes, and 45 cycles of 95°C for 0.30 minutes and 60°C for 1 minute. Results were obtained as threshold cycle (Ct) values and normalized gene expression with GAPDH. Data were analyzed based on 2-ΔΔCT method using CFX Manager software (Bio-Rad). For QPCR primers see **Table 1.**

**Table 1. OPCR Primers**

| **Gene Name** | **Forward Primer** | **Reverse Primer** |
|---|---|---|
| BCL2 | agtacctgaaccggcacct (SEQ ID NO: 1) | cagccaggagaaatcaaacag (SEQ ID NO: 2) |
| CD34 | gcgctttgcttgctgagttt (SEQ ID NO: 3) | gccatgttgagacacagggt (SEQ ID NO: 4 ) |
| TPO | ctcctcctgcttgtgacctc (SEQ ID NO: 5) | cccaagctaaagtccacagc (SEQ ID NO: 6) |
| P53 | tctgactgtaccaccaccatccacta (SEQ ID NO: 7) | caaacacgcacctcaaagc (SEQ ID NO:8) |
| NOTCH1 | gaggcgtggcagactatgc (SEQ ID NO: 19) | cttgtactccgtcagcgtga (SEQ ID NO: 10) |
| BMI1 | tggctctaatgaagatagagg (SEQ ID NO: 11) | ttccgatccaatctgttctg (SEQ ID NO: 12) |
| GATA1 | acaagatgaatgggcagaac (SEQ ID NO: 13) | tactgacaatcagcgcttc (SEQ ID NO: 14) |
| GATA2 | gatacccacctatccctcctatgtg (SEQ ID NO: 15) | gtggcaccacagttgacacactc (SEQ ID NO: 16) |
| HOXB4 | tcccactccgcgtgcaaaga (SEQ ID NO: 17) | gccggcgtaattggggttta (SEQ ID NO: 18) |
| P21 | gtcttgtacccttgtgcctc (SEQ ID NO: 19) | ggtagaaatctgtcatgctgg (SEQ ID NO: 20) |
| MPO | accctcatccaacccttc (SEQ ID NO: 21) | gtcaatgccaccttccag (SEQ ID NO: 22) |
| P27 | tttaattgggtctcaggcaaactct (SEQ ID NO: 23) | ccgtctgaaacattttcttctgttc (SEQ ID NO: 24) |
| C-MYC | tcctcggattctctgctctc (SEQ ID NO: 25) | cttgttcctcctcagagtcg (SEQ ID NO: 26) |
| HOXA9 | tctccttcgcgggcttg (SEQ ID NO: 27) | ccgacagcggttgaggtt (SEQ ID NO: 28) |
| PU1 | tgttacaggcgtgcaaaatggaagg (SEQ ID NO: 29) | ctcgtgcgtttggcgttggtataga (SEQ ID NO: 30) |
| GAPDH | tggaaggactcatgaccaca (SEQ ID NO: 31) | ttcagctcagggatgacctt (SEQ ID NO: 32) |

### Example 7

### Lentivirus production and transduction

Lentiviral particles were generated by transient co-transfection of 293T cells with the lentiviral vectors pLL3.7, pHR'8.9ΔVPR and pCMV-VSVG. CD34+ cells were infected in medium containing lentiviral particles and 8 µg/ml protamin. The culture medium was then removed and replaced with fresh media.

### Example 8

### Screening of compounds including epigenetic modifiers which increase CD34+CD90+ cells

Certain compounds such as TSA, TSA with 5 Azacytidine, Valproic acid, Chlamydocin, and trapoxinwere reported to expand CD34+CD90+ cells. To identify or confirm the molecules that expand CD34+CD90+ cells, the Inventors developed an assay using primary human CD34+ cells from PBSC and evaluated CD34 and CD90 expression by high throughput flow cytometry after a 4 or 5-day culture.

Using this assay, the Inventors screened FDA approved 446 compounds and 14 small molecule drugs including a panel of epigenetic modifiers as shown in **Table 2**. Human PBSC CD34+ cells were purified and cultured in IMDM with fetal bovine serum (FBS; GIBCO; final concentration 30%) and CC100 (a CD34 maintaining culture medium containing stem cell factor; SCF, Flt3 ligand; FL, Interleukin-3; IL3 and Interleukin-6; IL6; Stem Cell Technologies). The *ex vivo* culture approach is summarized in **Figure 8****.**

It was observed that TSA, DLS3, MS275, SAHA, UNC0638 or SRI expanded CD34+CD90+ population compared with that of DMSO treated CD34+ cells (**Figures 1A****,** **9** **and** **10**), while the survey of the FDA-approved 446 compounds yielded no potential hits for this purpose (Data not shown).

Because most of these compounds affected the total cell number of progeny, the absolute number of CD34+CD90+ cell was calculated. TSA, MS275 and JY1 expanded the absolute number of CD34+CD90+ cells more than that of other compounds (**Figure 1B**).

These findings suggested that the epigenetic modifiers, especially histone deacetylase inhibitors (HDACi) excluding HDAC6 inhibitor preferentially expanded CD34+CD90+ cells, while FDA approved 446 compounds did not expand CD34+CD90+ cells.

**Table 2. Epigenetic Modifiers in Asasy**

| Name | Function |
|---|---|
| TSA | pan HDAC inhibitor |
| DLSJ | HDAC112 inhibitor |
| MS275 | HDAC113 inhibitor |
| SAHA | pan HADC inhibitor: HDAC6 |
| 161 | HDAC6 inhibitor |
| 5-Azacytidine | hypomelhylating agent |
| JQ1-S | BET inhibitor |
| JY1 | DOT1 L inhibitor |
| UNC0638 | H3K9me2 methyltransferase inhibitor |
| JMJDJ | Histone HJ Lys 27 (H3K27) demethylase inhibitor |
| JQ-EZ-05 | EZH2 inhibitor; H3K27me3 methyltranferase inhibitor |
| SRI | Antagonist of the aryl hydrocarbon receptor |
| DBZ | Gamma Secretase inhibitor |
| dmPGE2 | stable prostaglandin E2 (PGE2) derivative |

### Example 9

### TSA or MS-275 in PBMC CD34+ cells leads to preferential expansion of CD34+CD90+ cells during ex vivo culture

To further investigate the role of TSA or MS-275 in expansion of PBSC CD34+ cells, TSA treatment led to a preferential expansion of CD34+CD90+ cells on day 3, day 5 and day7 compared with DMSO treated cells (p<.05), as shown in **Figure 11****,** **Figures 12B and F****.**

Similar results were seen in MS275 treated cell compared with DMSO-treated cells (p<.05). Conversely the growth rate with TSA and MS275 were about 2 times lower than that with DMSO **(****Figure 3A**; DMSO 10.7x 10⁴ ± 1.12 vs TSA 28.3 x 10⁴± 0.49; p<.05, vs MS275 16.7 x 10⁴ ± 1.06). By quantitating the absolute cell number of CD34+ **(****Figure 11****,** **Figures 12C and G****)**, and CD34+CD90+ **(****Figure 11****,** **Figures 12D and H****)** subpopulations, it was observed that absolute number of CD34+CD90+ cells was increased by either TSA or MS275 treatment from day 5 to day7.

To assess whether the lower total nucleated cell number observed in TSA treated culture was due to a delay in differentiation or proliferation, the Inventors performed a cell-division-monitoring dye carboxyfluorescein succinimidyl ester (CFSE) assay, a fluorescent cytoplasmic dye which allowed counting of cell divisions of CD34+ cells after *in vitro* culture. While 66.0 % of non-treated CD34+ cells divided at least more than 4 times by day 5, only 9.02% of TSA treated cells went through a similar number of cell divisions **(****Figure 3B****)**. Similar results were seen at day7 **(****Figure 3B**).

The Inventors then investigated whether TSA treatment led to increased CD34+CD90+ cells through self-renewal proliferation as assessed by surface markers. Using CD34+CD90-cells as a control population, the cell division profile of CD34+CD90+ cells upon TSA treatment was analyzed using the CSFE assay after 5 and 7 days of *in vitro* culture. Interestingly, while 31.4% of CD34+CD90+ cells were less divided, about 66.7% of the cells were more divided than that of CD34+CD90- cells by day5. Similar results were seen at day7 **(****Figure 3D****)**.

To investigate whether the lower total nucleated cell number observed in TSA treated culture was possibly due to apoptosis, annexin V staining with TSA ,MS275 or DMSO treated cells on day 3, 5 and 7 was performed. It was observed that 50nM TSA and 500nM MS275 did not significantly increased apoptotic cells **(****Figure 3E****).**

Taken together, the lower expansion rate of TSA treated CD34 cells appeared to result from slower cell division rather than induction of apoptosis. The described data indicates that TSA treatment induced self-renewal proliferation of CD34+CD90+ cells, albeit at a slower cell division rate and less tendency to differentiate.

### Example 10

### Treatment of CB CD34+ cells with TSA or MS-275 enhances the marrow-repopulating potential in vivo

Since the CD34+CD90+ cells are a CD34+ subpopulation with severe combined immunodeficient (SCID) repopulating activity (SRA), The Inventors next test whether the preferential expansion of these cells mediated by TSA or MS-275 treatment can lead to increased hematopoietic differentiation/proliferation in vitro and SRA engraftment *in vivo.* The Inventors first treated PBMC CD34+ cells with TSA or MS-275 treatments followed by assessment of *in vitro* colony formation in methylcellulose. Eight days after plating, the majority of TSA or MS275 treated colonies derived from CFU-GM were much larger than control group **(****Figure 13A****).** To quantify large and small colonies, The Inventors classified as large as >50 and small as 5-50 clusters, respectively. Whereas cytokines/growth factors treated cells generated high proportion of small colonies, TSA treated cells gave mainly rise to large colonies **(****Figure 13B****).** Fourteen days after plating, treatment of PBMC CD34+ cells resulted in similar distribution of colony-forming cell (CFC) content compared to other control groups **(****Figure 13C****)**, in which the majority of colonies were CFUGM despite of slight increased percentage of BFU-E.

During the culture of CB CD34+ cells with cytokines/growth factors and either cytokine alone or TSA , total cell numbers were dramatically increased from day 5 to 7. Based on *in vitro* phenotypic analysis described above, The Inventors transplanted cells after culture for 5 or7 days with cytokine alone or TSA into NSG mice to evaluate immunodeficient (SCID) repopulating activity (SRA) as a measure of enhanced HSPC function. Because Vanheuden et al, reported that severe combined immunodeficient (SCID) repopulating activity (rSRA) is mediated by CD34+CD38CD90+CD45RA- cells after *in vitro* culture, the Inventors first analyzed SRA activity in peripheral blood (PB) 2 weeks after transplant. The Inventors observed that mice transplanted with day 5 progeny of 2x10⁴ CB CD34+ cells treated with TSA had a higher percentage of human CD45+ engraftment (defined by ≥0.1% human CD45+ cells) compared with the progeny of CB CD34+ cells treated with cytokine (**Figure 4**).

### Example 11

### Treatment of PBMC CD34+ cells with TSA increased the efficiency of gene insertion approach

Next, the Inventors investigated whether expansion of CD34+CD90+ cells by TSA could affect the lentiviral transduction efficacy. A transfer vector encoding green fluorescent protein (GFP) was used for evaluation of lentiviral transduction. PBMC CD34+ cells were cultured with or without TSA for 3 days, followed by lentiviral transduction with protamin twice for 48 hours **(****Figure 15A**).

It was observed that percentage of CD34+GFP+ cells treated with TSA was higher than that of cells without treatment **(****Figure 15B and C****)**. Interestingly, the percentage of GFP positive cells was higher in CD34+CD90- cells compared with CD34+CD90+ cells **(****Figure 15B and D****)**. These data suggested that TSA promoted the lentiviral transduction for CD34+ cells *ex vivo.*

### Example 12

### Treatment of PBSC CD34+ cells with TSA modulates expression of stem cell related genes

Next, to investigate the molecular mechanism responsible for the expansion of functional HSCs and HSPC observed following TSA treatment, the Inventors examined expression levels of a number of genes involved in self-renewal or differentiation of stem cells using realtime PCR.

The Inventors observed higher levels of transcripts for GATA1, GATA2, HOXA9, HOXB4, PTEN, SALL4, p53, TPO and CD34 genes in cells treated with TSA **(****Figure 6**; p<.05), suggesting that these factors contributed to the augmented increased self-renewal property in PBSC CD34+ cells.

### Example 13

### Discussion

Hematopoietic cells that initiate engraftment in xenotransplants are operationally defined as Scidrepopulating cells (SRCs). This model provides a direct quantitative *in vivo* assay to measure human HSC activity and Lin-CD34+CD38-CD90+CD45RA- cord blood fractions are enhanced in SRC activity. On the other hand, it has been reported that the fresh stem cells lose self-renewal function after culture and an increase in CD34+CD38- cells does not correlate with SRC. Therefore, other HSC markers, such as CD90, have been utilized to confirm primitive HSPC candidate subpopulations in culture.

During *in vitro* culture of CD34+ cells with cytokines/growth factors, SCID repopulating cell activity was associated with CD90+ cells with no more than 2 divisions. Several investigators reported that primitive HSCs divide more slowly than relatively committed cells. While HSCs slowly cycle *in vivo,* in the presence of a variety of cytokine combinations, CD34+ cells rapidly cycle and undergo repeated cell divisions in culture, usually resulting in the generation of large number of differentiated progenitors but a decline in the number of marrow-repopulating cells.

Intriguingly, cord blood CD34+CD90+ cells exposed to epigenetic modulators such as 5-aza-2'-deoxycytidine (5azaD) and trichostatin (TSA) maintained assayable SRCs and possessed evidence of human multilineage hematopoietic cell engraftment even having after undergone 5 to 10 cell divisions in culture in the presence of cytokines/growth factors, This suggests that under the right *ex vivo* culture conditions, one might be able to expand the HSCs and/or HSPC without scarifying their stem cell properties. Several reports further showed that treatment of cord blood CD34+ cells with HDAC inhibitors induced histone H4 acetylation and expression of self-renewal genes in parallel with expansion of the CD34+CD90+ population.

The Inventors observed that the TSA slowly expanded CD34+CD90+ cells in culture and increased SRAs, in spite of a lower expansion rate of total nucleated cells as well as CD34+ cells compared to controls. These characteristics, expansion of CD34+CD90+ cells and slower division, are consistent with the intrinsic properties of primitive HSPCs. It has been reported that rapid severe combined immunodeficient (SCID) repopulating activity (rSRA) is mediated by CD34+CD38-CD90+CD45RA- cells in culture, and that long term SRC (LT-SRC) are restricted to CD34+CD90+ cells in culture cell. Although further investigation is necessary to understand the relationship among HSPC self-renewal, slower cell division, and HDACs, the described data suggests HDACi treatment of PBMC CD34+ treatment can lead to an expansion of primitive HSPCs with co-expression of CD90.

In searching of a potential mechanism of peptide mediated HSPC expansion, the Inventors have examined gene expression profiles. The expression of several key HSPC function related genes in PBMC CD34+ cells is affected by the treatment of TSA, Whereas GATA-1 drives the differentiation of hematopoietic progenitors into a subset of the blood cell lineages, GATA-2 is mainly expressed in hematopoietic stem and early progenitor cells and plays a pivotal role in self-renewal. This is likely one of the contributing factors that increase HSPC-like properties. Over expression HOXB4, member of homeobox family of genes, has been a potent stimulator of HSPC expansion. The greater expression of HOXB4 transcripts in CD34+ cells with TSA is consistent with the proposed role in HSPC self-renewal. The Inventors also compared our gene expression profile with those obtained form the treatment with 5Aaza/TSA. It has been reported that the increased expression levels of HOXB4, BMI1, GATA2, p21, p27, c-myc and MPO in the CB CD34+ cells treated with 5Aza/TSA. Without being bound by any particular, it is possible that transcriptional factors mediated CD34+CD90+ expansion when cultured *ex vivo* under a combination of HDACi and cytokines/growth factors (Fig.5).

The various methods and techniques described above provide a number of ways to carry out the invention. Of course, it is to be understood that not necessarily all objectives or advantages described may be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as may be taught or suggested herein. A variety of advantageous and disadvantageous alternatives are mentioned herein. It is to be understood that some preferred embodiments specifically include one, another, or several advantageous features, while others specifically exclude one, another, or several disadvantageous features, while still others specifically mitigate a present disadvantageous feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be mixed and matched by one of ordinary skill in this art to perform methods in accordance with principles described herein. Among the various elements, features, and steps some will be specifically included and others specifically excluded in diverse embodiments.

Although the invention has been disclosed in the context of certain embodiments and examples, it will be understood by those skilled in the art that the embodiments of the invention extend beyond the specifically disclosed embodiments to other alternative embodiments and/or uses and modifications and equivalents thereof.

Many variations and alternative elements have been disclosed in embodiments of the present invention. Still further variations and alternate elements will be apparent to one of skill in the art. Among these variations, without limitation, are the methods expanding hematopoietic cells, methods of modifying hematopoietic cells used in the described techniques, compositions of hematopoietic cells generated by the aforementioned techniques, treatment of diseases and/or conditions that relate to the teachings of the invention, techniques and composition and use of solutions used therein, and the particular use of the products created through the teachings of the invention. Various embodiments of the invention can specifically include or exclude any of these variations or elements.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a" and "an" and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventor for carrying out the invention. Variations on those preferred embodiments will become apparent to those of ordinary skill in the art upon reading the foregoing description. It is contemplated that skilled artisans can employ such variations as appropriate, and the invention can be practiced otherwise than specifically described herein. Accordingly, many embodiments of this invention include all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification. Each of the above cited references and printed publications are herein individually incorporated by reference in their entirety.

In closing, it is to be understood that the embodiments of the invention disclosed herein are illustrative of the principles of the present invention. Other modifications that can be employed can be within the scope of the invention. Thus, by way of example, but not of limitation, alternative configurations of the present invention can be utilized in accordance with the teachings herein. Accordingly, embodiments of the present invention are not limited to that precisely as shown and described.

Alternative expressions of the inventive concept are set out in the following clauses:
1. A method of expanding hematopoietic cells, comprising:
   (a) providing a quantity of hematopoietic cells; and
   (b) culturing the quantity of hematopoietic cells in the presence of at least one small molecule and at least one growth factor, wherein the at least one small molecule and at least one growth factor are capable of expanding the hematopoietic cells.
2. The method of clause 1, wherein the hematopoietic cells comprise hematopoietic stem cells (HSCs).
3. The method of clause 1, wherein the hematopoietic cells comprise hematopoietic stem progenitor cells (HSPCs).
4. The method of clause 1, wherein the hematopoietic cells are isolated from cord blood.
5. The method of clause 1, wherein the hematopoietic cells are isolated from bone marrow.
6. The method of clause 1, wherein the hematopoietic cells are isolated from peripheral blood.
7. The method of clause 1, wherein the at least one small molecule comprises a
   histone deacetylase inhibitor (HDACi).
8. The method of clause 7, wherein the HDACi comprises one or more HDACi
   selected from the group consisting of: trichostatin (TSA), DLS3, MS275, SAHA, and HDAC6 inhibitor161.
9. The method of clause 1, wherein the at least one small molecule comprises one or more small molecules selected from the group consisting of: 5-Azacytidine, JQ1-S, JY1, UNC0638, JMJD3, JQ-EZ-05, SRI, DBZ, dmPGE2 and UM171.
10. The method of clause 1, wherein the at least one growth factor comprises one or more growth factors elected from the group consisting of: stem cell factor (SCF), flt3 ligand (FL),interleukin-3 (IL3) and interleukin-6 (IL6).
11. A method of genomic editing comprising:
   (a) providing a quantity of hematopoietic cells; and
   (b) culturing the quantity of hematopoietic cells in the presence of at least one small molecule and at least one growth factor;
   (c) contacting the cells with one or more vectors, each vector encoding at least one selection cassette and/or at least one nuclease; and
   (d) selecting for hematopoietic cells expressing the selection cassette and the nuclease, wherein cells expressing the selection cassette and the nuclease comprise an edited genome.
12. The method of clause 11, wherein the cells comprise hematopoietic stem cells (HSCs).
13. The method of clause 11, wherein the pluripotent stem cells comprise hematopoietic stem progenitor cells (HSPCs).
14. The method of clause 11, wherein the at least one nuclease comprises a Zinc Finger Nuclease (ZFN).
15. The method of clause 11, wherein the at least one nuclease comprises a Transcription Activator-Like Effector Nuclease (TALENs).
16. The method of clause 11, wherein the at least one nuclease comprises a CRISPR-associated protein (Cas) nuclease.
17. The method of clause 11, wherein the one or more vector comprises a vector encoding at least one selection cassette and at least one nuclease.
18. The method of clause 11, wherein the quantity of hematopoietic cells are isolated from cord blood, bone marrow, or peripheral blood.
19. The method of clause 18, comprising:
   (e) administering the selected hematopoietic cells into a subject.
20. The method of clause 19, wherein the hematopoietic cells are immunocompatible with the subject.
21. A quantity of cells produced by the method of clause 11.
22. An *ex vivo* method of expanding hematopoietic cells, comprising:
   (a) obtaining a quantity of hematopoietic stem cells (HSCs) or hematopoietic stem progenitor cells (HSPCs) from cord blood, bone marrow, or peripheral blood; and
   (b) expanding the HSCs or HSPCs by culturing the HSCs or HSPCs in the presence of:
      (i) at least one small molecule selected from the group consisting of: trichostatin (TSA), DLS3, MS275, SAHA, HDAC6 inhibitor161, 5-Azacytidine, JQ1-S, JY1, UNC0638, JMJD3, JQ-EZ-05, SRI, DBZ, dmPGE2 and UM171; and
      (ii) at least one growth factor selected from the group consisting of: stem cell factor (SCF), flt3 ligand (FL),interleukin-3 (IL3) and interleukin-6 (IL6), for a period of at least 48 hours, thereby expanding the HSCs or HSPCs.
23. The method of clause 22, wherein obtaining a quantity of HSPCs comprises isolation of cells that express CD34+ and/or CD90+.
24. The method of clause 22, wherein the period of at least 48 hours comprises 72 hours, 96 hours, 120 hours, 144 hours, or 168 hours.

## Claims

1. A method of genetically modifying a quantity of expanded hematopoietic cells, comprising:
a. providing a quantity of hematopoietic cells;
b. culturing the quantity of hematopoietic cells in the presence of at least one histone deacetylase inhibitor (HDACi) and at least one growth factor to expand the hematopoietic cells; and
c. contacting the expanded hematopoietic cells with a nucleic acid sequence,
wherein the nucleic acid sequence modifies the quantity of expanded hematopoietic cells.

2. The method of claim 1, wherein the hematopoietic cells comprise hematopoietic stem cells (HSCs).

3. The method of claim 1, wherein the hematopoietic cells comprise hematopoietic stem progenitor cells (HSPCs).

4. The method of claim 1, wherein the hematopoietic cells are isolated from cord blood, bone marrow or peripheral blood.

5. The method of claim 1, wherein the nucleic acid sequence is comprised within a vector.

6. The method of claim 1, wherein the at least one HDACi is selected from the group consisting of: trichostatin (TSA), MS275, SAHA, Valproic acid, and HDAC6 inhibitor WT161.

7. The method of claim 1, wherein the at least one growth factor is selected from the group consisting of: stem cell factor (SCF), flt3 ligand (FL), interleukin-3 (IL3) and interleukin-6 (IL6)

8. The method of claim 1, further comprising in step (b) further culturing the quantity of hematopoietic cells in the presence of one or more small molecules selected from the group consisting of: JQ1-S, JY1, UNC0638, JMJD3, JQ-EZ-05, SRI, DBZ, dmPGE2 and UM171.

9. The method of claims 1, wherein the nucleic acid sequence encodes a nuclease.

10. The method of claim 8, wherein the nuclease is selected from the group consisting of: a Zinc Finger Nuclease (ZFN), a Transcription Activator-Like Effector Nuclease (TALENs), and a CRISPR-associated protein (Cas) nuclease.

11. The method of claim 1, wherein the nucleic acid sequence is comprised within a lentivirus.

12. The method of claim 1, wherein contacting is transfection or transduction.

13. The method of claim 1, further comprising the step of selecting for the modified expanded hematopoietic cells that express the nucleic acid sequence.

14. A composition comprising the quantity of expanded hematopoietic cells of claim 1.

15. The composition of claim 13, further comprising a pharmaceutically acceptable carrier.


SEQUENCE LISTING

<110>  THE BRIGHAM AND WOMEN'S HOSPITAL, INC.
       DANA-FARBER CANCER INSTITUTE, INC.
       NATIONAL UNIVERSITY OF SINGAPORE
       CHAI, Li
       FEDERATION, Alexander
       TATETSU, Hiro
       TENEN, Daniel G.
       BRADNER, James

<120>  COMPOSITIONS AND METHODS FOR EX VIVO EXPANSION OF HUMAN
       HEMATOPOIETIC STEM/PROGENITOR CELLS

<130>  043214-081011

<150>  61/970,787
<151>  2014-03-26

<160>  32

<170>  PatentIn version 3.5

<210>  1
<211>  19
<212>  DNA
<213>  Homo sapiens

<400>  1
agtacctgaa ccggcacct                                                    19


<210>  2
<211>  21
<212>  DNA
<213>  Homo sapiens

<400>  2
cagccaggag aaatcaaaca g                                                 21


<210>  3
<211>  20
<212>  DNA
<213>  Homo sapiens

<400>  3
gcgctttgct tgctgagttt                                                   20


<210>  4
<211>  20
<212>  DNA
<213>  Homo sapiens

<400>  4
gccatgttga gacacagggt                                                   20


<210>  5
<211>  20
<212>  DNA
<213>  Homo sapiens

<400>  5
ctcctcctgc ttgtgacctc                                                    20


<210>  6
<211>  20
<212>  DNA
<213>  Homo sapiens

<400>  6
cccaagctaa agtccacagc                                                    20


<210>  7
<211>  23
<212>  DNA
<213>  Homo sapiens

<400>  7
tctgactgta ccaccatcca cta                                                23


<210>  8
<211>  19
<212>  DNA
<213>  Homo sapiens

<400>  8
caaacacgca cctcaaagc                                                     19


<210>  9
<211>  19
<212>  DNA
<213>  Homo sapiens

<400>  9
gaggcgtggc agactatgc                                                     19


<210>  10
<211>  20
<212>  DNA
<213>  Homo sapiens

<400>  10
cttgtactcc gtcagcgtga                                                    20


<210>  11
<211>  21
<212>  DNA
<213>  Homo sapiens

<400>  11
tggctctaat gaagatagag g                                                  21


<210>  12
<211>  20
<212>  DNA
<213>  Homo sapiens

<400>  12
ttccgatcca atctgttctg                                                   20


<210>  13
<211>  20
<212>  DNA
<213>  Homo sapiens

<400>  13
acaagatgaa tgggcagaac                                                   20


<210>  14
<211>  19
<212>  DNA
<213>  Homo sapiens

<400>  14
tactgacaat cagcgcttc                                                    19


<210>  15
<211>  25
<212>  DNA
<213>  Homo sapiens

<400>  15
gatacccacc tatccctcct atgtg                                             25


<210>  16
<211>  23
<212>  DNA
<213>  Homo sapiens

<400>  16
gtggcaccac agttgacaca ctc                                               23


<210>  17
<211>  20
<212>  DNA
<213>  Homo sapiens

<400>  17
tcccactccg cgtgcaaaga                                                   20


<210>  18
<211>  20
<212>  DNA
<213>  Homo sapiens

<400>  18
gccggcgtaa ttgggtttta                                                   20


<210>  19
<211>  20
<212>  DNA
<213>  Homo sapiens

<400>  19
gtcttgtacc cttgtgcctc                                                   20


<210>  20
<211>  21
<212>  DNA
<213>  Homo sapiens

<400>  20
ggtagaaatc tgtcatgctg g                                                 21


<210>  21
<211>  18
<212>  DNA
<213>  Homo sapiens

<400>  21
accctcatcc aacccttc                                                     18


<210>  22
<211>  18
<212>  DNA
<213>  Homo sapiens

<400>  22
gtcaatgcca ccttccag                                                     18


<210>  23
<211>  25
<212>  DNA
<213>  Homo sapiens

<400>  23
tttaattggg tctcaggcaa actct                                             25


<210>  24
<211>  25
<212>  DNA
<213>  Homo sapiens

<400>  24
   ccgtctgaaa cattttcttc tgttc                                              25


<210>  25
<211>  20
<212>  DNA
<213>  Homo sapiens

<400>  25
tcctcggatt ctctgctctc                                                   20


<210>  26
<211>  20
<212>  DNA
<213>  Homo sapiens

<400>  26
cttgttcctc ctcagagtcg                                                   20


<210>  27
<211>  17
<212>  DNA
<213>  Homo sapiens

<400>  27
tctccttcgc gggcttg                                                      17


<210>  28
<211>  18
<212>  DNA
<213>  Homo sapiens

<400>  28
ccgacagcgg ttgaggtt                                                     18


<210>  29
<211>  25
<212>  DNA
<213>  Homo sapiens

<400>  29
tgttacaggc gtgcaaaatg gaagg                                             25


<210>  30
<211>  25
<212>  DNA
<213>  Homo sapiens

<400>  30
ctcgtgcgtt tggcgttggt ataga                                             25


<210>  31
<211>  20
<212>  DNA
<213>  Homo sapiens

<400>  31
tggaaggact catgaccaca                                                   20


<210>  32
<211>  20
<212>  DNA
<213>  Homo sapiens

<400>  32
ttcagctcag ggatgacctt                                                   20